(19) Europäisches Patentamt
European Patent Office
Office européen des brevets

(11) EP 4 331 615 A1

(12) **EUROPEAN PATENT APPLICATION**
published in accordance with Art. 153(4) EPC

(43) Date of publication:
**06.03.2024 Bulletin 2024/10**

(21) Application number: **22795032.6**

(22) Date of filing: **29.04.2022**

(51) International Patent Classification (IPC):
***A61K 47/68*** (2017.01) ***C07K 16/28*** (2006.01)
***A61P 35/00*** (2006.01)

(52) Cooperative Patent Classification (CPC):
**A61K 47/65; A61K 47/68; A61P 35/00; A61P 35/02; C07K 5/1005; C07K 16/00; C07K 16/28; C12N 5/10; C12N 15/85;** Y02A 50/30

(86) International application number:
**PCT/CN2022/090450**

(87) International publication number:
**WO 2022/228563 (03.11.2022 Gazette 2022/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.04.2021 CN 202110481199**

(71) Applicants:
- **Jiangsu Mabwell Health Pharmaceutical R&D Co., Ltd.**
  **China Medical City**
  **Taizhou, Jiangsu 225300 (CN)**
- **Mabwell (Shanghai) Bioscience Co., Ltd.**
  **Shanghai 201210 (CN)**

(72) Inventors:
- **ZHOU, Wei**
  **Shanghai 201210 (CN)**
- **TAN, Xiaoding**
  **Shanghai 201210 (CN)**
- **LIU, Datao**
  **Shanghai 201210 (CN)**

(74) Representative: **Schiweck Weinzierl Koch Patentanwälte Partnerschaft mbB**
**Ganghoferstraße 68 B**
**80339 München (DE)**

Remarks:
The complete document including Reference Table(s) and the Sequence Listing(s) can be downloaded from the EPO website

(54) **ANTIBODY-DRUG CONJUGATE TARGETING NECTIN-4 AND PREPARATION METHOD THEREFOR AND USE THEREOF**

(57) An antibody-drug conjugate targeting a poliovirus receptor-like molecule 4 (Nectin-4). The antibody-drug conjugate can be used for preparing a drug for treating Nectin-4-related diseases. The antibody-drug conjugate has strong targeting properties to Nectin-4 and a strong endocytosis effect via the target, and has an excellent tumor-killing effect.

EP 4 331 615 A1

## Description

## CROSS-REFERENCE TO RELATED APPLICATION(S)

The present patent application claims the benefit of priority of Chinese Patent Application No. CN202110481199.4 filed on 30 April 2021, the content of which is hereby incorporated by reference in its entirety.

## TECHNICAL FIELD

The invention relates to an antibody-drug conjugate, in particular to an antibody-drug conjugate targeting poliovirus receptor-like molecule 4 (Nectin-4) and a preparation method and use thereof.

## BACKGROUND OF THE INVENTION

Nectins (poliovirus receptor-like molecules) are a novel class of cell adhesion proteins, which regulate cell-cell adhesions either cooperatively with or independently of cadherins. Nectins comprises a family of four members, Nectin-1, -2, -3, and -4. All the nectins have one extracellular region with three Ig-like loops, one transmembrane segment and one cytoplasmic tail. Among those members, Nectin-4 is specifically expressed in embryo and placenta as well as tumor cells, and has been found in some studies to be closely related to the generation and development of a variety of tumor cells. For example, an analysis on pathological sections derived from 2394 tumor patients revealed that Nectin-4 was widely expressed in the patient population suffering from bladder cancer, breast cancer and pancreatic cancer. Therefore, Nectin-4 has become an important target for the diagnosis and treatment of many tumors or cancers.

Antibody-drug conjugate (ADC) technology is one that precisely delivers antitumor drugs (e.g., cytotoxic agents, cytostatic agents, small-molecule chemotherapeutic agents, etc.) to target tumor cells through utilizing the ability of antibodies to specifically recognize specific antigens on the surface of the tumor cells, and accumulates and intracellularly releases the antitumor drugs, thereby precisely killing tumors. An antibody-drug conjugate is generally composed of three parts: an antibody or antibody-like ligand, a small molecule drug, and a linker coupling the two together. Antibody-drug conjugates have been considered as one of the most promising antineoplastic drugs due to their appropriate molecular weights, high stability, strong targeting property, and small toxic and side effects.

However, there are a number of issues must be considered and addressed for successfully developing an ADC. For example, the antibody is required to specifically recognize the diseased region, has low allergenicity, and can be efficiently and rapidly internalized via endocytosis; the linker which couples the antibody and the drug, shall be highly stable in blood and needs to be specifically activated and efficiently release the small molecule drugs in the targeted cells, otherwise would produce an unacceptable level of toxicity to normal cells; and the coupled small molecule drug needs to have strong cell killing activity and the like. In the antibody-drug conjugates currently in clinical trials, high-activity cytotoxic small molecule drugs are typically coupled via linkers to either lysine residues on the antibody surface or cysteine residues in the hinge region, with an optimal drug-to-antibody ratio (DAR) of 2-4. However, the large number of lysine residues (more than 80) on the antibody surface and the non-selectivity of the conjugation reaction lead to an uncertain number of coupled drugs and uncertain conjugation sites, which in turn leads to the production of non-uniform antibody-drug conjugates. For example, antibody-drug conjugate T-DM1 made from the targeted medicine Trastuzumab (anti-HER2 antibody) and the microtubule inhibitor DM1 (derivative of maytansine) has a DAR distribution of 0-8 (with an average DAR of 3.5). Similarly, although there are only four interchain disulfide bonds in the hinge region of an antibody, partial reduction of the interchain disulfide bonds is required to achieve an optimal average DAR (2-4). However, currently available reducing agents (DTT, TCEP, etc.) cannot selectively reduce the interchain disulfide bonds, and thus the resulting conjugates are not uniform, but are composed of a plurality of components. Among the components, the main components have a DAR of 0, 2, 4, 6, or 8, and the components having one corresponding specific DAR occur in isomers which are formed due to different conjugation sites. Heterogeneity of ADC products may lead to heterogeneity of pharmacokinetic properties, potency, and toxicity, among the components. For example, components with higher DAR are cleared more rapidly in vivo and result in higher toxicity.

Seattle Genetics, cooperated with Astellas, provided an anti-Nectin-4 antibody-drug conjugate named as Enfortumab Vedotin (Padcev) utilizing their unique linker mc-vc-MMAE and anti-Nectin-4 antibody Enfortumab via random conjugation. The results of one clinical trial showed that in the patients receiving chemotherapy and PD-1/PD-L1 inhibitor regimens, the patients receiving Enfortumab Vedotin exhibited a medium overall survival of 12.9 months, 3.9 months longer than that of the patients receiving chemotherapy in the control group, thereby showing a good efficacy for tumor treatment by Enfortumab Vedotin. However, clinical studies had also found that the Enfortumab Vedotin treatment is often accompanied by fever, skin itching, peripheral neuropathy, dry eyes, neutrocytopenia, etc. These adverse reactions are directly associated with the excessive coupling of the small molecule to the antibody and the unstable coupling way.

Therefore, there is still an urgent need for an efficient, simple, and practical chemical conjugation method for

the research and development of an antibody-drug conjugate targeting Nectin-4.

## SUMMARY OF THE INVENTION

The technical problem to be solved by the invention is to provide an antibody which is capable of specifically binding to human Nectin-4 with high affinity using hybridoma screening and humanization technologies, by which the antibody will have the fewest number of murine amino acids through humanization design and in turn have better in-vivo safety and application prospect; and on the basis of the antibody, to further screen an antibody that has a stronger internalization effect, and the antibody will be prepared into an antibody-drug conjugate with a small-molecule chemical drug. The antibody-drug conjugate will combine the antibody's targeting to Nectin-4 expressing cells and strong internalization effect via the target Nectin-4, with the small-molecule chemical drug's actions, to achieve an excellent tumor killing effect.

Accordingly, one object of the present disclosure is to provide an antibody or fragment thereof capable of specifically binding to Nectin-4. In the context of the disclosure, the fragment of an antibody encompasses various functional fragments of the antibody, for example, an antigen-binding portions thereof, such as an Fab, F(ab')2, or scFv fragment. Another object of the present disclosure is to provide an antibody-drug conjugate targeting Nectin-4 or salt thereof, which is prepared using the antibody or fragment thereof.

The present disclosure provides the following technical solutions.

In one aspect, the present disclosure provides an antibody-drug conjugate targeting Nectin-4 or salt thereof, comprising an anti-Nectin-4 antibody or fragment thereof covalently linked to a drug.

In the antibody-drug conjugate or salt thereof provided by the present disclosure, the anti-Nectin-4 antibody or fragment thereof comprises a heavy chain and a light chain comprising heavy chain complementarity determining regions 1 to 3 (CDR-H1, CDR-H2 and CDR-H3) and light chain complementarity determining regions 1 to 3 (CDR-L1, CDR-L2 and CDR-L3) respectively as follows :

(i) CDR-H1, CDR-H2 and CDR-H3 having amino acid sequences as shown in SEQ ID NO: 11, SEQ ID NO: 12 and SEQ ID NO: 13 respectively; and, CDR-L1, CDR-L2 and CDR-L3 having amino acid sequences as shown in SEQ ID NO: 14, SEQ ID NO: 15 and SEQ ID NO: 16 respectively;
(ii) CDR-H1, CDR-H2 and CDR-H3 having amino acid sequences as shown in SEQ ID NO: 11, SEQ ID NO: 17 and SEQ ID NO: 13 respectively; and, CDR-L1, CDR-L2 and CDR-L3 having amino acid sequences as shown in SEQ ID NO: 18, SEQ ID NO: 15 and SEQ ID NO: 16 respectively; or
(iii) CDR-H1, CDR-H2 and CDR-H3 having amino acid sequences as shown in SEQ ID NO: 19, SEQ ID NO: 20 and SEQ ID NO: 13 respectively; and, CDR-L1, CDR-L2 and CDR-L3 having amino acid sequences as shown in SEQ ID NO: 14, SEQ ID NO: 15 and SEQ ID NO: 16 respectively.

Preferably, the antibody-drug conjugate or salt thereof provided by the present disclosure has a formula as follows: Ab-[L-CTD]m, in which Ab represents the anti-Nectin-4 antibody or fragment thereof, L represents a linker, CTD represents the drug, and m represents the average number of the drug coupled per one Ab molecule.

Preferably, in the antibody-drug conjugate or salt thereof provided by the present disclosure, CTD is a cytotoxic drug; preferably, CTD is one or more selected from the group consisting of: microtubule inhibitors MMAE, DM1, DM4, Tublysin, amanitin, calicheamicin, Eribulin and derivatives thereof; topoisomerase inhibitors SN38, Exatecan and derivatives thereof; and DNA binding agents PBD, doxorubicin and derivatives thereof.

m is 1.0 to 5.0, preferably 3.0 to 4.2, more preferably 3.5 to 4.5, still more preferably 3.8 to 4.2, yet more preferably 3.9 to 4.1, and particularly preferably 4.0.

Preferably, in the antibody-drug conjugate or salt thereof provided by the present disclosure, the heavy chain and the light chain comprised in the anti-Nectin-4 antibody or fragment thereof comprise a heavy chain variable region (VH) and a light chain variable region (VL) respectively, wherein the heavy chain variable region (VH) comprises an amino acid sequence as shown in SEQ ID NO: 1, SEQ ID NO: 3, SEQ ID NO: 5 or SEQ ID NO: 7 or a variant thereof, and the light chain variable region (VL) comprises an amino acid sequence as shown in SEQ ID NO: 2, SEQ ID NO: 4, SEQ ID NO: 6 or SEQ ID NO: 8 or a variant thereof.

More preferably, in the antibody-drug conjugate or salt thereof provided by the present disclosure, the heavy chain variable region (VH) and the light chain variable region (VL) comprised in the anti-Nectin-4 antibody or fragment thereof comprise respectively:

(i) the amino acid sequence as shown in SEQ ID NO: 1 or a variant thereof; and, the amino acid sequence as shown in SEQ ID NO: 2 or a variant thereof;
(ii) the amino acid sequence as shown in SEQ ID NO: 3 or a variant thereof; and, the amino acid sequence as shown in SEQ ID NO: 4 or a variant thereof;

(iii) the amino acid sequence as shown in SEQ ID NO: 5 or a variant thereof; and, the amino acid sequence as shown in SEQ ID NO: 6 or a variant thereof; or

(iv) the amino acid sequence as shown in SEQ ID NO: 7 or a variant thereof; and, the amino acid sequence as shown in SEQ ID NO: 8 or a variant thereof.

In the context of the present disclosure, a "variant" of an amino acid sequence refers to an amino acid sequence having at least 75% sequence identity (any percent identity greater than or equal to 75%, e.g., at least 80%, preferably at least 85%, more preferably at least 90%, further preferably at least 91%, 92%, 93%, 94%, 95%, 96%, 97%, 98%, or even 99% identity, etc.) to the amino acid sequence.

In particular, the anti-Nectin-4 antibody or fragment thereof according to the present disclosure comprises at least a heavy chain variable region and a light chain variable region, both comprising CDRs as above and interspersed Framework Regions (FRs), which domains are arranged as follows: FR1-CDR1-FR2-CDR2-FR3-CDR3-FR4. Thus, with respect to the heavy chain variable region and the light chain variable region comprised in the anti-Nectin-4 antibody or fragment thereof provided by the present disclosure, the up to 25% difference due to the "at least 75% sequence identity" may be present in any framework region in the heavy chain variable region or the light chain variable region. Or, with respect to the anti-Nectin-4 antibody or fragment thereof according to the present disclosure as a whole, the up to 25% difference may be present in any domain or sequence in the antibody or fragment thereof according to the present disclosure other than the heavy chain variable region and the light chain variable region. The difference may be resulted from amino acid deletion, addition or substitution at any position, and the substitution may be conservative substitution or non-conservative substitution.

In the antibody-drug conjugate or salt thereof provided by the present disclosure, the anti-Nectin-4 antibody or fragment thereof is in any form, e.g., a monoclonal antibody, a single chain antibody, a diabody, a single domain antibody, a nanobody, a fully or partially humanized antibody, or a chimeric antibody and the like against Nectin-4; alternatively, the antibody or fragment thereof is a half-antibody or an antigen-binding fragment of the half-antibody against Nectin-4, e.g., single-chain variable fragment (scFv), bivalent single-chain variable fragment (BsFv), disulfide-stabilized variable fragment (dsFv), (disulfide-stabilized variable fragment)$_2$ ((dsFv)$_2$), antigen-binding fragment (Fab), Fab' fragment (Fab'), F(ab')$_2$ fragment (F(ab')$_2$), or variable fragment (Fv). With respect to the fragment provided by the present disclosure, preferably, the fragment is any fragment of the antibody capable of binding to mammal Nectin-4, preferably primate Nectin-4, more preferably human Nectin-4.

Preferably, in the antibody-drug conjugate or salt thereof provided by the present disclosure, the anti-Nectin-4 antibody or fragment thereof may further comprise a constant region. Preferably, the anti-Nectin-4 antibody or fragment thereof further comprises a human or murine heavy chain constant region (CH) and/or light chain constant region (CL), more preferably a heavy chain constant region selected from the group consisting of constant regions of IgG, IgA, IgM, IgD and IgE and/or a kappa or lambda type light chain constant region.

Preferably, the antibody is a monoclonal antibody, preferably a murine, chimeric, or humanized monoclonal antibody; more preferably, the heavy chain constant region of the monoclonal antibody is of IgG1 or IgG4 subtype and the light chain constant region of the monoclonal antibody is of kappa type. Alternatively, for example, the antibody is an immunoglobulin, in particular IgA, IgD, IgE, IgG or IgM, e.g., a human subtype of IgA, IgD, IgE, IgG or IgM, more preferably a human IgG1, IgG2, IgG3 or IgG4 subtype.

According to one particular embodiment of the present disclosure, the anti-Nectin-4 antibody or fragment thereof comprises a heavy chain constant region comprising the amino acid sequence as shown in SEQ ID NO: 9 or a variant thereof. Alternatively, the anti-Nectin-4 antibody or fragment thereof comprises a light chain constant region comprising the amino acid sequence as shown in SEQ ID NO: 10 or a variant thereof. As defined above, a "variant" of an amino acid sequence refers to an amino acid sequence having at least 75% sequence identity to the amino acid sequence.

Further, the antibody-drug conjugate or salt thereof provided by the present disclosure has a structure represented by the formula Ia and/or Ib as follows:

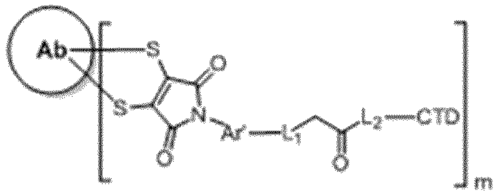

Ia

and/or

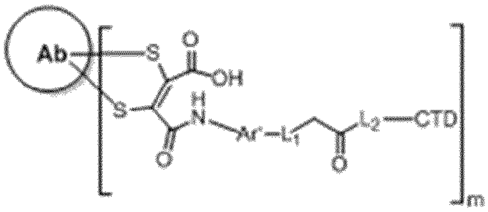

Ib

wherein:

Ab is the anti-Nectin-4 antibody or fragment thereof;

Ar' is any one selected from the group consisting of: substituted or unsubstituted C6-C10 arylene and substituted or unsubstituted 5-12 membered heteroarylene, wherein the substitution refers to the replacement of a hydrogen atom on a group by one or more substituents selected from the group consisting of: halogen (F, Cl, Br or I), halogenated alkyl (e.g. halogenated C1-C6 alkyl, preferably halogenated C1-C4 alkyl, e.g. trifluoromethyl) and alkoxy (e.g. C1-C6 alkoxy, preferably C1-C4 alkoxy, e.g. methoxy);

$L_1$ is -O(CH2CH2O)n- linked to Ar', wherein n is any integer in the range from 1 to 24, preferably from 1 to 10, more preferably from 3 to 5;

$L_2$ is an enzyme cleavable fragment, e.g., a dipeptide or a tripeptide or a tetrapeptide or a combination thereof with a cleavable self-immolating linker (i.e., a polypeptide fragment consisting of 2-4 amino acids, or a combination of the polypeptide fragment with a cleavable self-immolating linker), such as Val-Ala, Val-Ala-PAB, Val-Cit, Val-Cit-PAB, Phe-Lys-PAB, Ala-Ala-Ala, Gly-Gly-Phe-Gly (GGFG), MAC glucuronide phenol.

Preferably, $L_2$-CTD is VcMMAE, GGFG-Dxd or VC-seco-DUBA.

Preferably, in case that Ar' is a substituted or unsubstituted 5-12 membered heteroarylene, the heteroatom is N.

Preferably, Ar' is a substituted or unsubstituted C6 arylene or a substituted or unsubstituted 6 membered heteroarylene.

According to particular embodiments of the present disclosure, the antibody-drug conjugate or salt thereof provided by the present disclosure has the structure as follows:

Conjugate ADC-1:

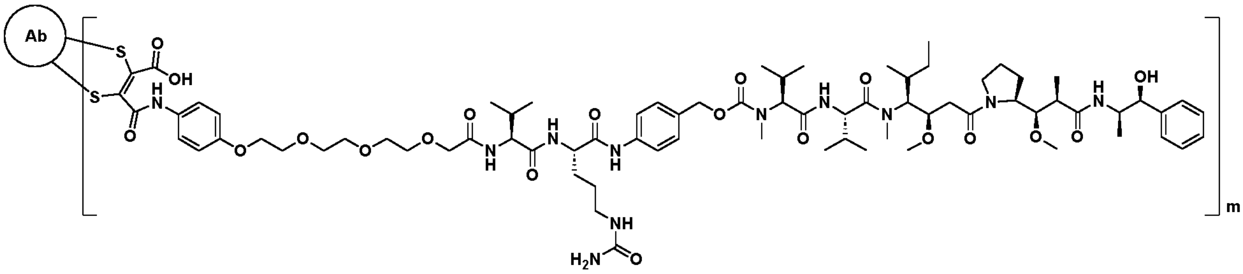

Conjugate ADC-2:

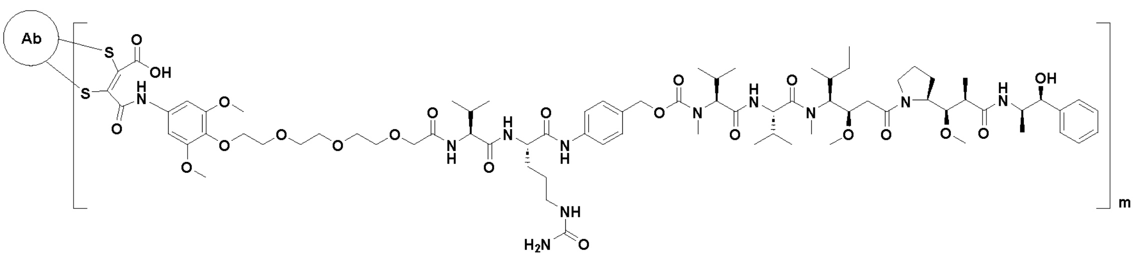

Conjugate ADC-3 :

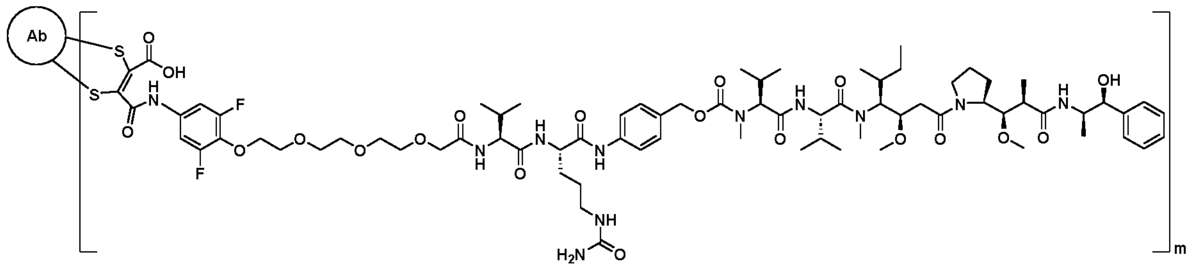

Conjugate ADC-4:

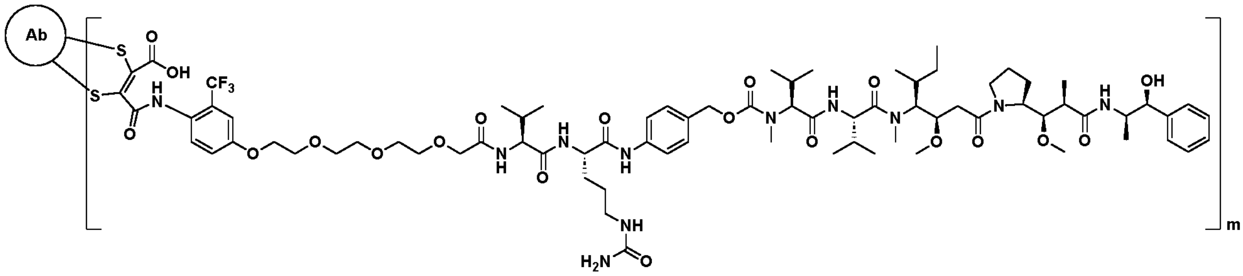

Conjugate ADC-5:

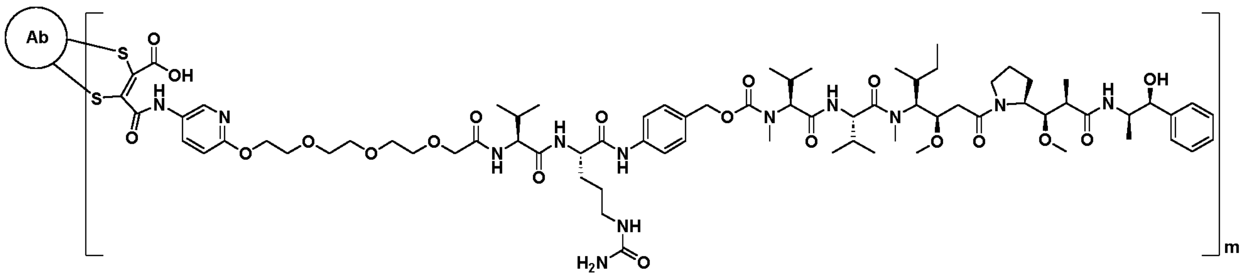

Conjugate ADC-6:

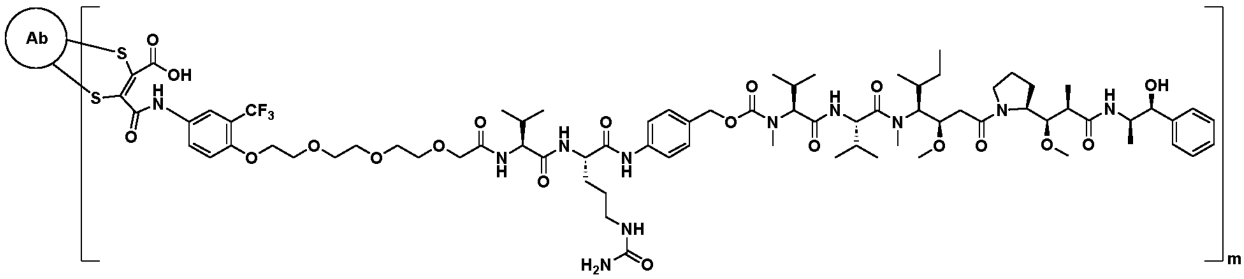

Conjugate ADC-7:

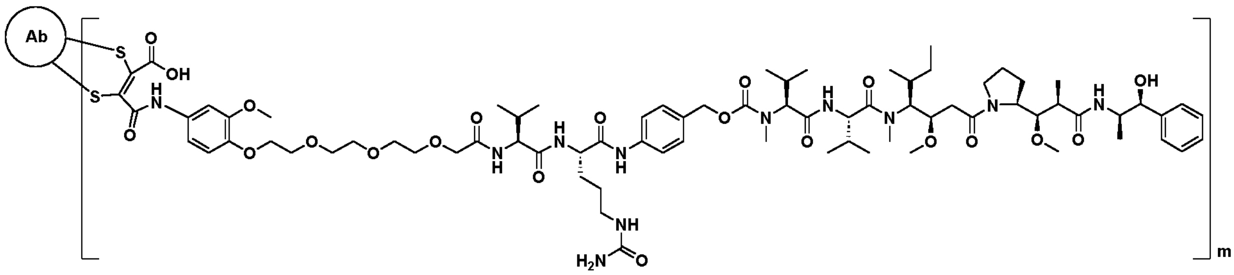

In another aspect, the present disclosure provides a preparation method for an antibody-drug conjugate targeting Nectin-4 or salt thereof, wherein the antibody-drug conjugate or salt thereof has a structure represented by the formula Ia and/or Ib as follows:

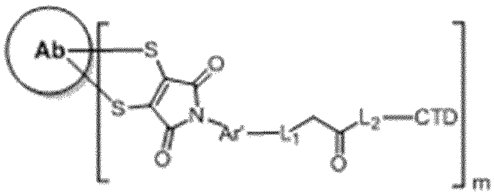

Ia

and/or

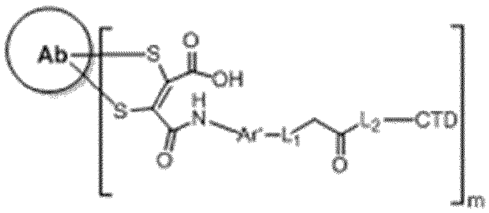

Ib

the method comprising the following steps:

(1) reacting the anti-Nectin-4 antibody or fragment thereof with a reducing agent in a buffer, to obtain a reduced antibody or fragment thereof;
(2) conjugating a drug-linker (a linker-drug conjugate) to the reduced antibody or fragment thereof obtained in step (1) in a mixture of a buffer and an organic solvent, to obtain the antibody-drug conjugate targeting Nectin-4 or salt thereof.

In the preparation method provided by the present disclosure, the anti-Nectin-4 antibody or fragment thereof is as defined above; the drug-linker has a structure represented by the formula Ic as follows:

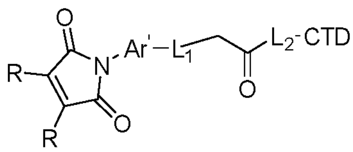

Ic

wherein:

R is X or R'S, wherein X is halogen (F, Cl, Br or I), preferably Br or I; R' is substituted or unsubstituted C6-C10 aryl or substituted or unsubstituted 5-12 membered heteroaryl, wherein the substitution refers to the replacement of a hydrogen atom on a group by one or more substituents selected from the group consisting of: alkyl (e.g., C1-C6 alkyl, preferably C1-C4 alkyl), alkoxy (e.g., C1-C6 alkoxy, preferably C1-C4 alkoxy, more preferably methoxy), halogen (F, Cl, Br or I), ester, amide and cyano;
preferably, R is R'S, wherein R' is phenyl or substituted phenyl, and the substituent in the substituted phenyl is selected from the group consisting of alkyl (e.g., C1-C6 alkyl, preferably C1-C4 alkyl), alkoxy (e.g., C1-C6 alkoxy, preferably C1-C4 alkoxy, more preferably methoxy), halogen (F, Cl, Br or I), ester, amide and cyano; preferably, R' is phenyl, 4-methylformamido-substituted phenyl

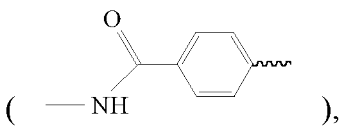

or 4-formylmorpholine-substituted phenyl

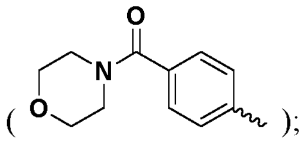

and

Ar', $L_1$, $L_2$ and CTD are as defined above.

In the context of the present disclosure, "drug-linker", "[L-D]", "drug-containing linker", "linker-drug conjugate" and the like are used interchangeably. According to particular embodiments of the application, the drug-linker is any one selected from the group consisting of:

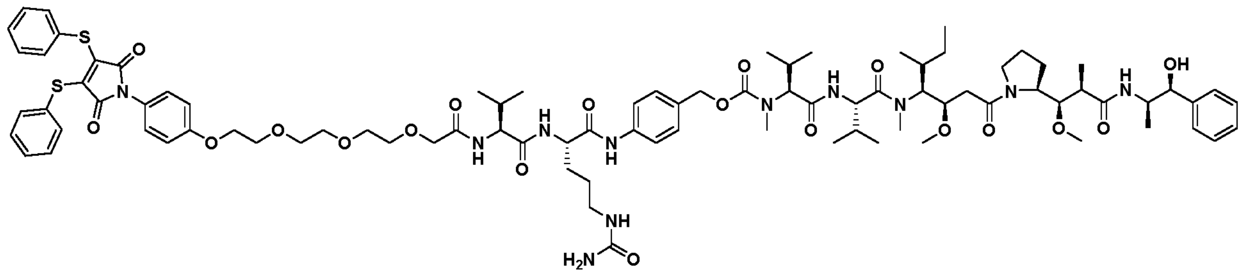

A-1

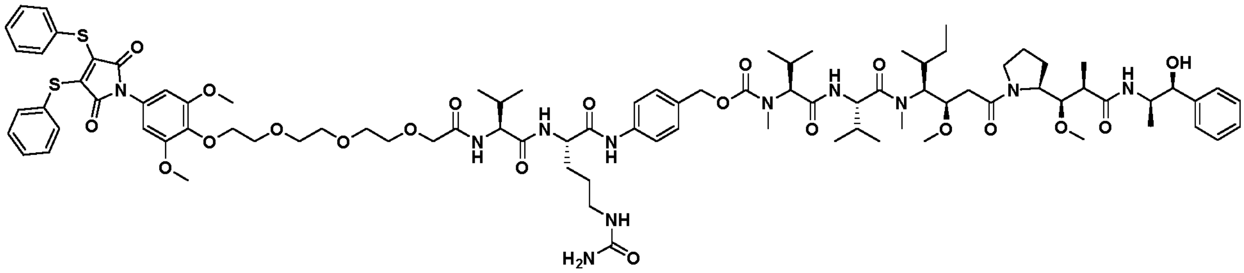

A-2

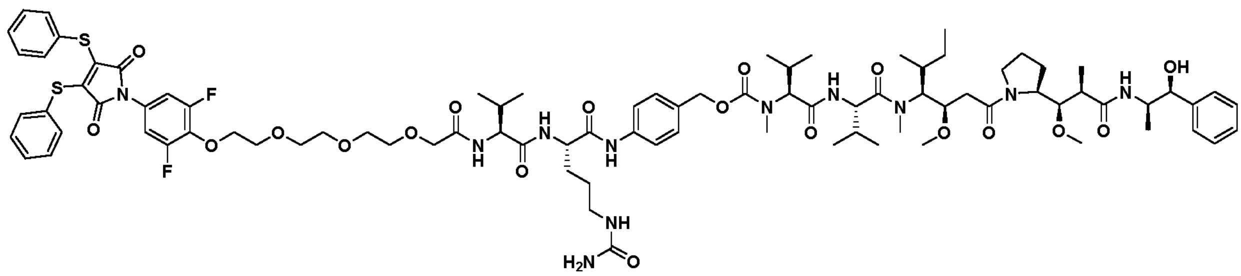

A-3

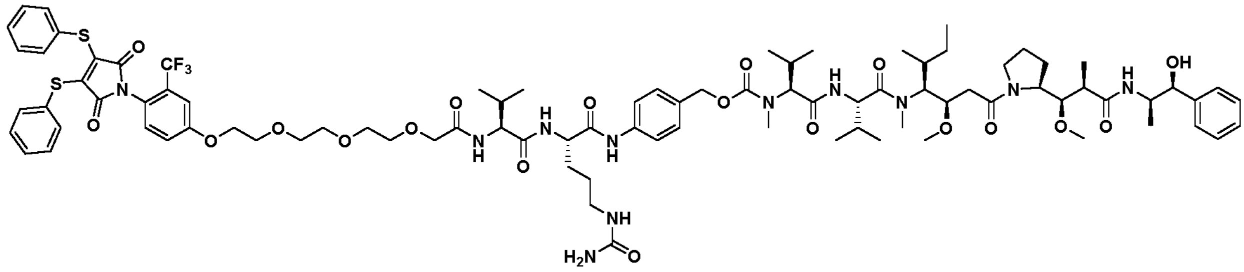

A-4

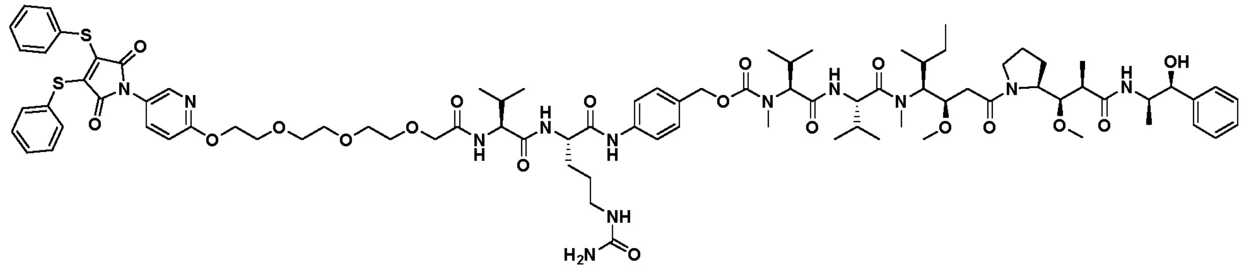

A-5

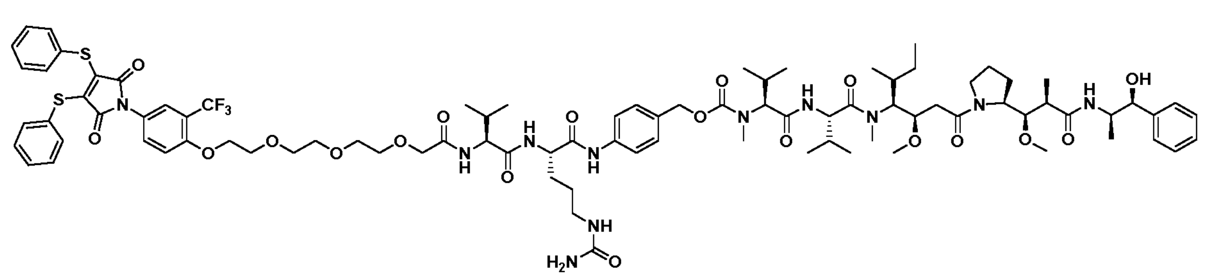

A-6

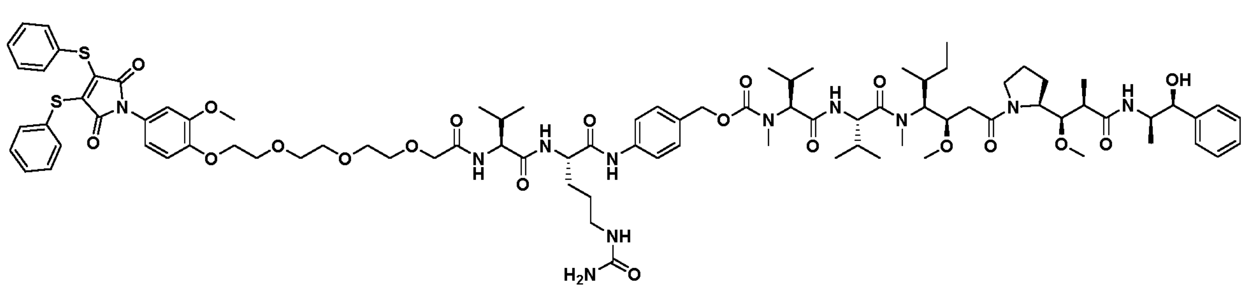

A-7

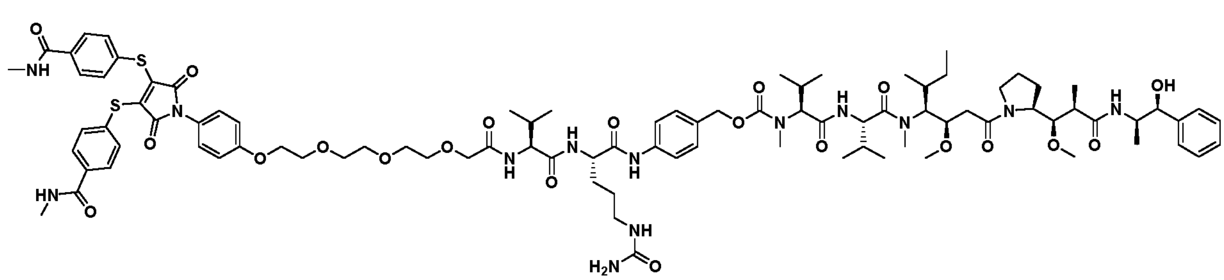

B-1

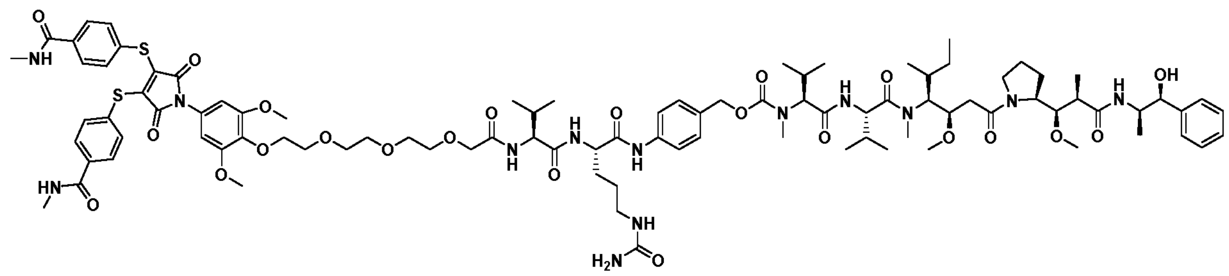

B-2

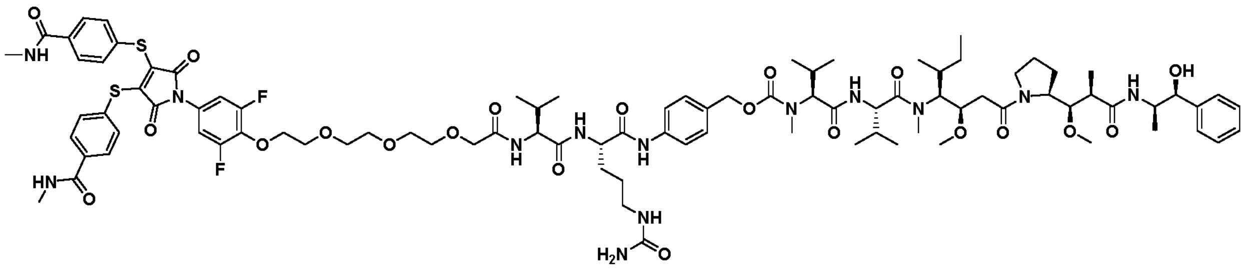

B-3

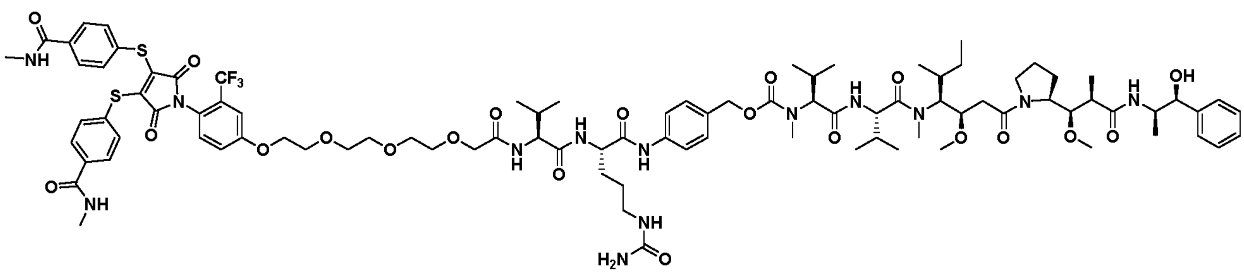

B-4

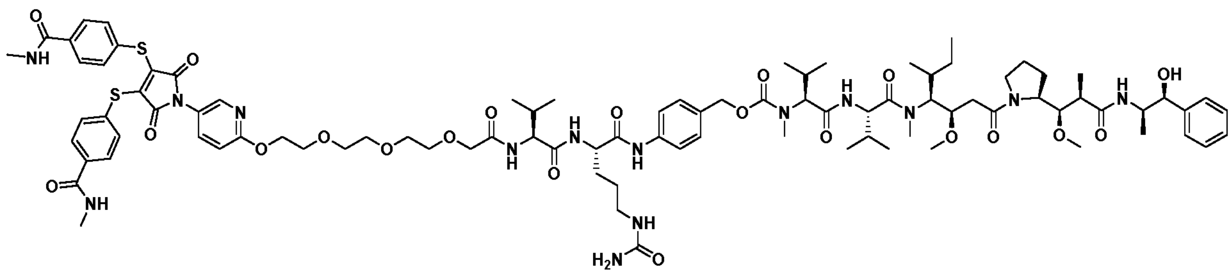

B-5

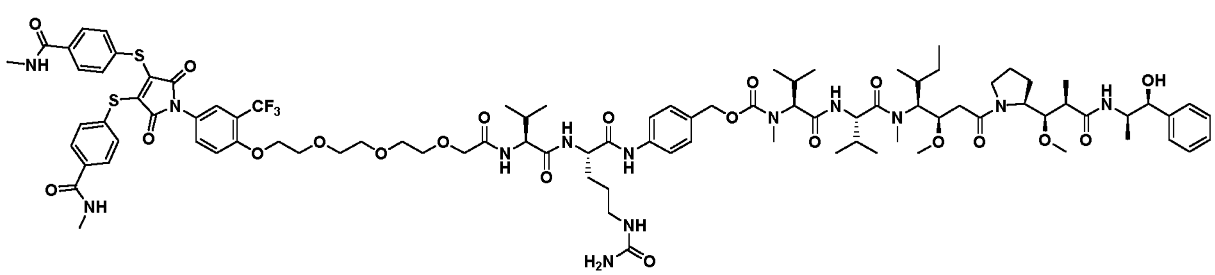

B-6

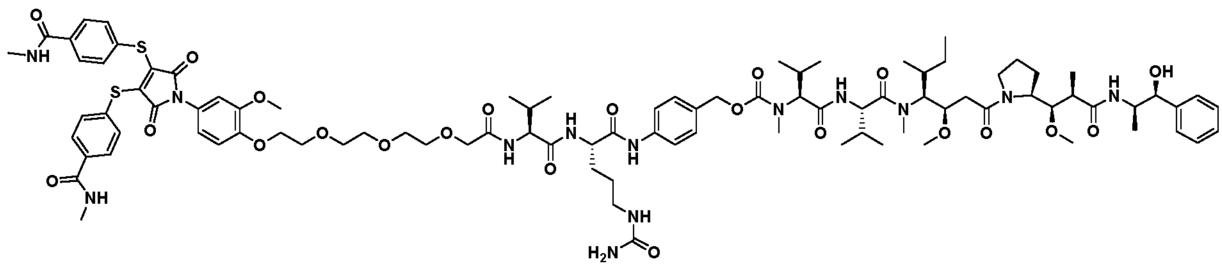

B-7

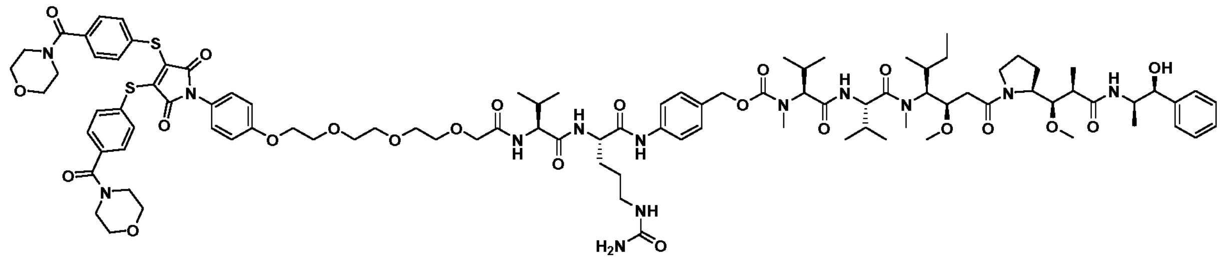

C-1

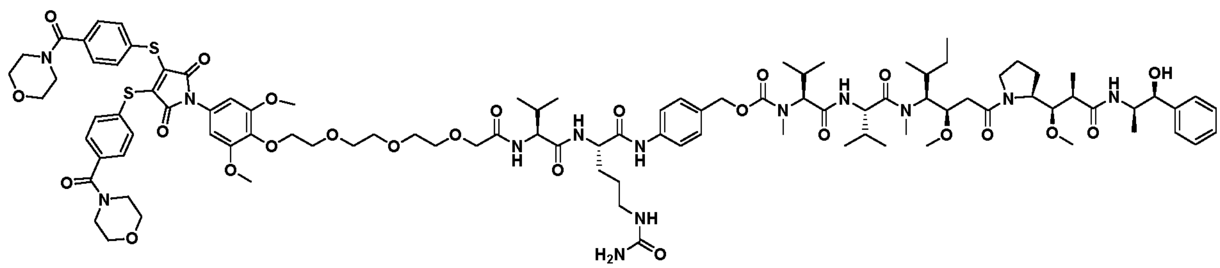

C-2

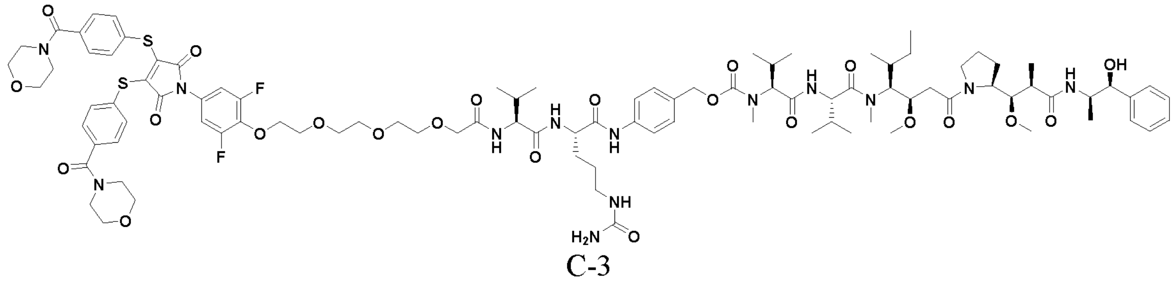

C-3

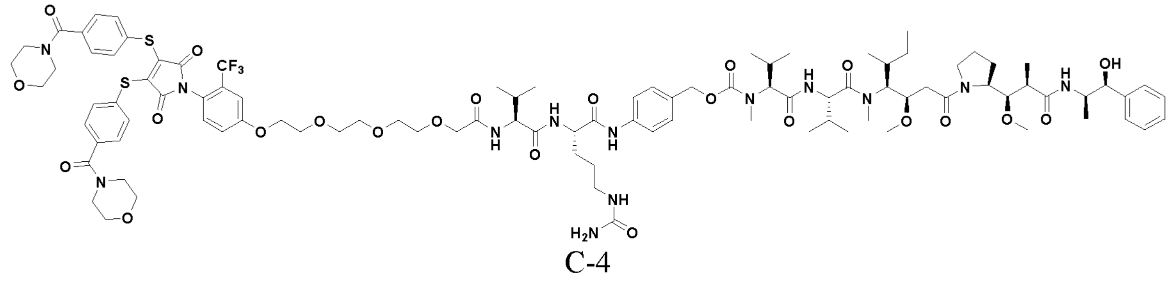

C-4

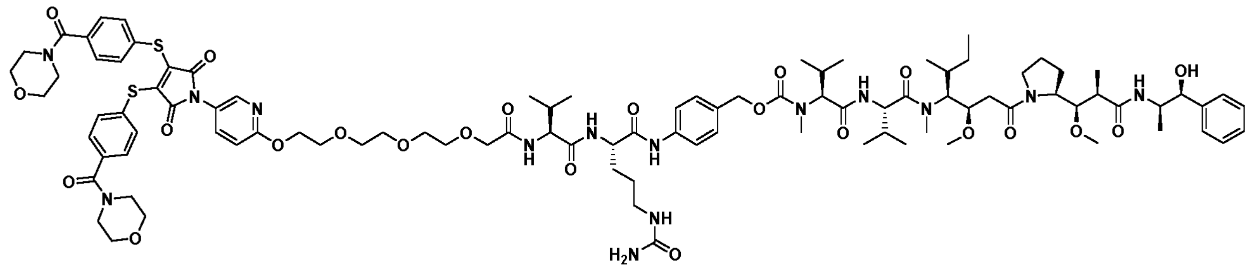

C-5

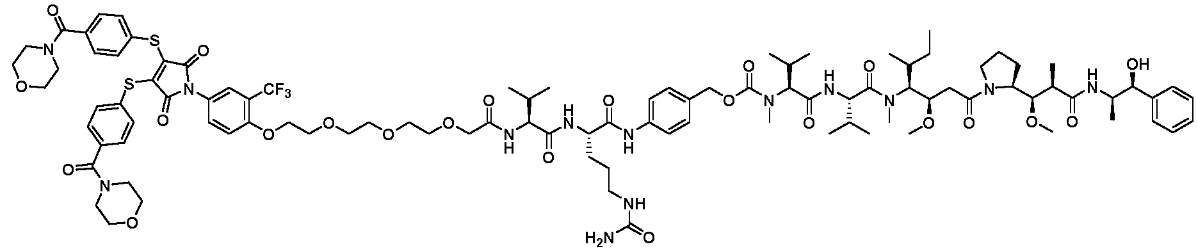

C-6

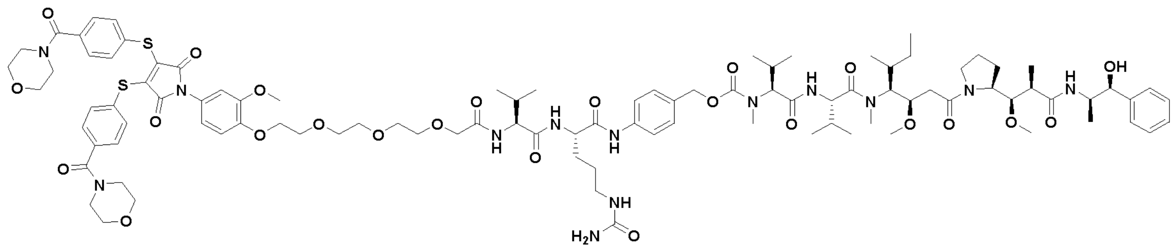

C-7

Preferably, the preparation method provided by the present disclosure comprises the following steps:

a. Antibody reduction: adding a reducing agent to a phosphate buffer containing the antibody in a concentration of 5-30 mg/mL at an equivalent molar ratio of ≥ 5.5: 1 (the reducing agent: the antibody), and reacting the reducing agent and the antibody for 1.5-2 hours, wherein the reducing agent is one or more selected from the group consisting of TCEP, DTT, 2-MEA, and DTBA;
b. Antibody conjugation: displacing the reduced antibody obtained in step a into a phosphate buffer at pH 6.5-7.8, thereby diluting the antibody to a concentration of 3.5-15 mg/mL in the buffer to obtain a diluted antibody solution; adding a drug-containing linker dissolved in an organic co-solvent to the diluted antibody solution at an equivalent molar ratio of 4.5-6.5: 1 (the drug-containing linker: the antibody), and then reacting the reaction system under stirring at 15-35 °C for ≥ 0.5 hours, wherein the organic co-solvent is one or more selected from the group consisting of DMA, DMSO, DMF, and ACN;
c. Hydrophobic chromatography: subjecting the antibody conjugation product obtained to purification through hydrophobic chromatography using hydrophobic filler.

Further preferably, the preparation method further comprises the following step after step b or after step c:
d. Hydrolysis: displacing the antibody conjugation product into a phosphate buffer at pH 7.4-9.0, and heating the buffer at 35±10 °C for 2-24 hours, to obtain a hydrolysis product.
Preferably, the displacement in steps a, b and d is performed using molecular sieves, ultracentrifuge tubes or ultrafiltration membranes. Preferably, the hydrophobic filler used in step c may be Butyl Sepharose HP, Capto Phenyl Impres, or Butyl Sepharose FF.
In yet another aspect, the present disclosure provides an anti-Nectin-4 antibody or fragment thereof. The description and definitions with respect to the anti-Nectin-4 antibody or fragment thereof comprised in the antibody-drug conjugate targeting Nectin-4 or salt thereof as above apply to the anti-Nectin-4 antibody or fragment thereof in this aspect. With respect to sequences in detail, the anti-Nectin-4 antibody or fragment thereof provided by the present

disclosure comprises a heavy chain and a light chain comprising heavy chain complementarity determining regions 1 to 3 (CDR-H1, CDR-H2 and CDR-H3) and light chain complementarity determining regions 1 to 3 (CDR-L1, CDR-L2 and CDR-L3) respectively as follows:

CDR-H1, CDR-H2 and CDR-H3 having amino acid sequences as shown in SEQ ID NO: 19, SEQ ID NO: 20 and SEQ ID NO: 13 respectively; and, CDR-L1, CDR-L2 and CDR-L3 having amino acid sequences as shown in SEQ ID NO: 14, SEQ ID NO: 15 and SEQ ID NO: 16 respectively.

Preferably, the heavy chain and the light chain comprised in the anti-Nectin-4 antibody or fragment thereof comprise a heavy chain variable region (VH) and a light chain variable region (VL) respectively, wherein the heavy chain variable region (VH) comprises an amino acid sequence as shown in SEQ ID NO: 5 or SEQ ID NO: 7 or a variant thereof, and the light chain variable region (VL) comprises an amino acid sequence as shown in SEQ ID NO: 6 or SEQ ID NO: 8 or a variant thereof.

More preferably, the heavy chain variable region (VH) and the light chain variable region (VL) comprised in the anti-Nectin-4 antibody or fragment thereof comprise:

(A) the amino acid sequence as shown in SEQ ID NO: 5 or a variant thereof; and, the amino acid sequence as shown in SEQ ID NO: 6 or a variant thereof; or

(B) the amino acid sequence as shown in SEQ ID NO: 7 or a variant thereof; and, the amino acid sequence as shown in SEQ ID NO: 8 or a variant thereof.

Further in this aspect, the anti-Nectin-4 antibody or fragment thereof may also have the respective characteristics as defined herein above for the antibody part in the antibody-drug conjugate.

According to the antibody provided by the present disclosure as above, the present disclosure provides a nucleic acid molecule comprising a nucleotide sequence encoding a heavy chain variable region, a light chain variable region, a heavy chain or a light chain comprised in the anti-Nectin-4 antibody or fragment thereof according to the present disclosure.

The nucleic acid molecule according to the present disclosure can be cloned into a vector which in turn transfects or transforms a host cell. Accordingly, in yet another aspect, the present disclosure provides a vector comprising the nucleic acid molecule of the present disclosure. The vector can be a eukaryotic expression vector, a prokaryotic expression vector, an artificial chromosome, a phage vector and the like.

The vector or nucleic acid molecule provided by the present disclosure may be used to transform or transfect a host cell or in any way enter a host cell for antibody preservation or expression, etc. Thus, in a further aspect, the present disclosure provides a host cell comprising the nucleic acid molecule and/or the vector of the present disclosure, or transformed or transfected with the nucleic acid molecule and/or the vector of the present disclosure. The host cell may be any prokaryotic or eukaryotic cell, such as a bacterial or insect, fungus, plant or animal cell.

The anti-Nectin-4 antibody or fragment thereof provided by the present disclosure can be obtained using any conventional techniques known in the art. For example, the heavy chain variable region and/or the light chain variable region of the antibody or the heavy chain and/or the light chain of the antibody may be obtained from the nucleic acid molecule provided by the present disclosure, and then they can be assembled with optional other domains of the antibody to obtain the antibody; alternatively, the host cell provided by the present disclosure is cultured under conditions that allow the host cell to express the heavy chain variable region and/or the light chain variable region of the antibody or the heavy chain and/or the light chain of the antibody and assemble them into the antibody. Optionally, the method further includes a step of recovering the produced antibody.

The antibody-drug conjugate targeting to Nectin-4 or salt thereof, the anti-Nectin-4 antibody or fragment thereof, the nucleic acid molecule, the vector, or the host cell may be contained in a composition, more particularly in a pharmaceutical preparation, to be used for various purposes as actually needed. Thus, in still a further aspect, the present disclosure also provides a composition comprising the antibody-drug conjugate targeting to Nectin-4 or salt thereof, the anti-Nectin-4 antibody or fragment thereof, the nucleic acid molecule, the vector, and/or the host cell of the present disclosure. Preferably, the composition is a pharmaceutical composition which optionally comprises a pharmaceutically acceptable carrier, accessory material or excipient.

In still another aspect, the present disclosure provides use of the antibody-drug conjugate targeting to Nectin-4 or salt thereof, the anti-Nectin-4 antibody or fragment thereof, the nucleic acid molecule, the vector, the host cell, and/or the composition in the manufacture of a medicament for treating a tumor. Alternatively, the present disclosure provides a method for treating a tumor, comprising administering to a subject in need thereof the antibody-drug conjugate targeting to Nectin-4 or salt thereof, the anti-Nectin-4 antibody or fragment thereof, the nucleic acid molecule, the vector, the host cell, and/or the composition. The subject is a mammal, preferably a primate, further preferably a human.

Preferably, the tumor is a tumor or cancer associated with high expression of Nectin-4; preferably, the tumor or cancer is any one selected from the group consisting of: bladder cancer, breast cancer, ovarian cancer, pancreatic cancer, hepatocellular cancer, gastric cancer, non-hodgkin's lymphoma, hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, prostate cancer, colorectal cancer, glioma, and mesothelioma.

## BRIEF DESCRIPTION OF THE DRAWINGS

Embodiments of the invention are described in detail below with reference to the attached figures, in which:

Fig. 1 shows the characterization results of ADC 3d, in which panel 1A: Hydrophobic Interaction Chromatography (HIC) profile; 1B: Size Exclusive Chromatography (SEC) profile; 1C: Non-Reducing Capillary Electrophoresis-Sodium Dodecyl Sulfate (NR-CE-SDS) profile; 1D: Liquid Chromatography Mass Spectrometry (LCMS) spectrum.

Fig. 2 shows the results of in vivo efficacy of different ADCs.

Fig. 3 shows the results of in vivo efficacy of different ADCs.

Fig. 4 shows the results of in vivo efficacy of different ADCs.

Fig. 5 shows the results of in vivo efficacy of different ADCs.

Fig. 6 shows the results of in vivo efficacy of different ADCs.

## DETAILED DESCRIPTION OF PREFERRED EMBODIMENTS

The invention is illustrated below with reference to specific examples. It will be understood by those skilled in the art that these examples are merely illustrative of the invention and do not limit the scope of the invention in any way.

Experimental procedures in the following examples are all conventional ones, unless otherwise specified. Raw materials and reagents used in the following examples are all commercially available products, unless otherwise specified.

**Group 1 of Examples** Screening and preparation of anti-Nectin-4 antibodies

Mice were immunized with a recombinant human Nectin-4 protein (purchased from Novoprotein Scientific Inc., Catalog No.: CJ19), and B cells were obtained from the immunized mice and fused with SP20 myeloma cells previously prepared to screen positive hybridoma cells capable of binding to human Nectin-4 antigen. Positive hybridoma cell strains each secreting only one antibody were finally obtained.

After expanded culture of the hybridoma cells secreting anti-Nectin-4 antibodies, total RNA was extracted from the cells and then reversely transcribed into cDNA. The sequences of light chain variable region IgVL (κ) and heavy chain variable region VH of the antibodies were amplified by PCR. PCR products were purified and ligated to a T vector. The obtained vectors were transformed into E. coli cells. After extension culture of the cells, plasmids were extracted for DNA sequencing to obtain sequences of the heavy and light chain variable regions of the monoclonal antibodies.

The heavy chain variable region sequence of each murine anti-human Nectin-4 monoclonal antibody and the heavy chain constant region sequence of published human monoclonal antibody IgG1 subclass (SEQ ID NO: 9) were spliced together and constructed into a mammalian cell expression vector; and the light chain variable region sequence of each murine anti-human Nectin-4 monoclonal antibody and the light chain constant region sequence of published human monoclonal antibody κ subclass (SEQ ID NO: 10) were spliced together and constructed into a mammalian cell expression vector. The constructed heavy chain and light chain vectors for preparing anti-human Nectin-4 chimeric antibodies were mixed in pairs, HEK293 cells were transfected with the vectors using Polyethyleneimine (PEI), and cell supernatants were collected about 7 days later. Anti-human Nectin-4 chimeric antibodies were obtained using Mabselect.

According to a comprehensive analysis with antibody coding schemes, amino acid sequences of 6 complementarity determining regions (CDRs) and framework regions supporting the conserved three-dimensional conformation of the heavy and light chains of each murine antibody were determined. Subsequently, the heavy chain variable region sequence of human antibody which mostly resembles to the murine antibody was searched from known human antibody sequences, and IGITVI | IGHJ4*01, for example, was selected. The framework region sequences in it were selected as a template, and the heavy chain CDRs of the murine antibody were combined with the framework regions of the human antibody, and a humanized heavy chain variable region sequence was ultimately produced. In the same manner, a humanized light chain variable region sequence was produced. According to the changes in binding activity, individual amino acids in the framework regions were subjected to back mutation, changing from human ones to murine ones, and/or individual amino acids were modified if post-translation modification occurred. Alternative humanized heavy chain

variable regions and light chain variable regions were finally obtained.

The humanized heavy chain variable regions and light chain variable regions were combined in pairs, and humanized antibodies were obtained by reference to the preparation process of chimeric antibodies as above. Through in vitro cell-binding assay against human Nectin-4, endocytosis assay in target cancer cells, target cancer cell proliferation inhibition assay, as well as through measuring the antibodies' antigen-binding abilities, internalization activities or pharmacodynamics upon being prepared into ADCs with known drug-containing linkers, and the like, the humanized antibodies as follows were screened (with CDRs underlined):

**42D20-hz63**

VH (SEQ ID NO: 1; CDRs sequentially: SEQ ID NO: 11, SEQ ID NO: 12, SEQ ID NO: 13, according to CHOTHIA numbering scheme) QVQLQESGPGLVKPSETLSLTCTVSGFSLIDYGVSWIRQPPGKGKLEWIGVIWGGGKIYYNSVLKSRVTISKDNSKSQVSLKLSSVTAADTAVYYCAKQGGLLFYAMDYWGQGTLVTVSS VL (SEQ ID NO: 2; CDR sequentially: SEQ ID NO: 14, SEQ ID NO: 15, SEG ID NO: 16, according to CHOTHIA numbering scheme)

DIVMTQSPDSLAVSLGERATINCKSSQSLLNTYSQKNYLAWYQQKPGQSPKLLIYFASTRESGVPDRFSGSGSETDFTLTISSLQAEDLAVYFCQQHYNTPFTFGAGTKLELK

**42D20-hz10:**

VH (SEQ ID NO: 3; CDR sequentially: SEQ ID NO: 11, SEQ ID NO: 17, SEQ ID NO: 13, according to CHOTHIA numbering scheme) QVQLQESGPGLVKPSETLSLTCTVSGFSLIDYGVSWIRQPPGKGLEWIGVIWGDGKIYYNSVLKSRVTISKDNSKSQVSLKLSSVTAADTAVYYCAKQGGLLFTAMDYWGQGTLVTVSS VL (SEQ ID NO: 4: CDR sequentially: SEQ ID NO: 18, SEQ ID NO: 15, SEQ ID NO 16, according to CHOTHIA numbering scheme)

DIVMTQSPDSLAVSLGERATINCKSSQSLLNSYSQKNYLAWYQQKPGQPPKLLIYFASTRESGVPDRFSGSGSGTDFTLTISSLQAEDVAVYYCQQHYNTPFTFGAGTKLELK

**hH2L1:**

VH (SEQ ID NO: 5; CDR sequentially: SEQ ID NO: 19, SEQ ID NO: 20, SEQ ID NO: 13, according to KABAT numbering scheme) EVQLQESGPGLVKPSETLSLTCTVSGFSLIDYGVSWIRQPPGKGLEWIGVIWGGGKIYYNSVLKSRVTISKDNSKSQVSLKLSSVTAADTAVYYCAKQGGLLFYAMDYWGQGTLVTVSS VL (SEG ID NO: 6; CDR sequentially: SEQ ID NO: 14, SEG ID NO: 15, SEQ ID NO: 16, according to KABAT numbering scheme)

DIVMTQSPDSLAVSLGERATINCKSSQSLLNTYSQKNYLAWYQQKPGQPPKLLIYFASTRESGVPDRFSGSGSGTDFTLTISSLQAEDVAVYYCQQHYNTPFTFGGGTKVEIK

**hL2H1mut1:**

VH (SEQ ID NO: 7; CDR sequentially: SEQ ID NO: 19, SEQ ID NO: 20, SEQ ID NO: 13, according to KABAT numbering scheme) EVQLQESGPGLVKPSETLSLTCTVSGFSLIDYGVSWIRQPPGKGLEWIGVIWGGGKIYYNSVLKSRVTISVDTSKNQFSLKLSSVTAADTAVYYCAKQGGLLFYAMDYWGQGTLVTVSS VL (SEQ ID NO: 8: CDR sequentally: SEQ ID NO: 14, SEQ ID NO: 15, SEQ ID NO: 16, according to KABAT numbering scheme)

DIVMTQSPDSLAVSLGERATINCKSSQSLLNTYSQKNYLAWYQQKPGQSPKLLIYFASTRESGVPDRFSGSGSETDFTLTISSLQAEDLAVYFCQQHYNTPFTFGGGTKVEIK

**Group 2 of Examples** Preparation and physicochemical characterization of antibody-drug conjugates

The following methods are used for illustrative experiments:

**2.1** Preparation of drug-containing linkers C-1, C-2, C-3, and C-4

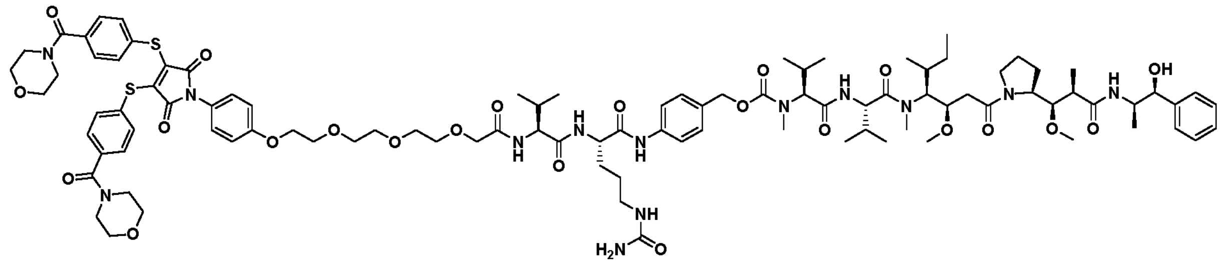

C-1

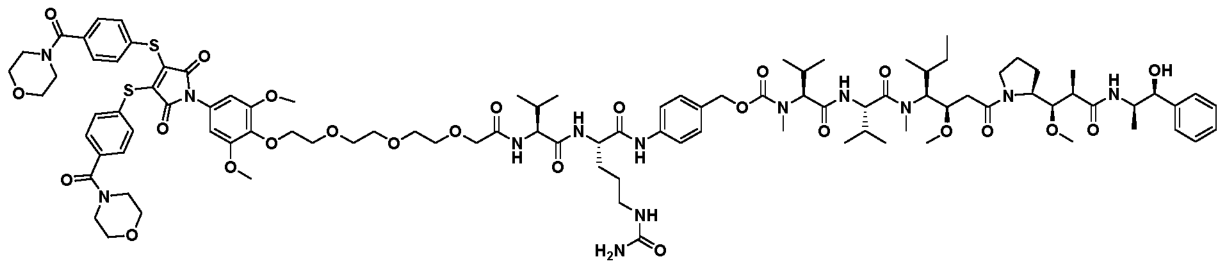

C-2

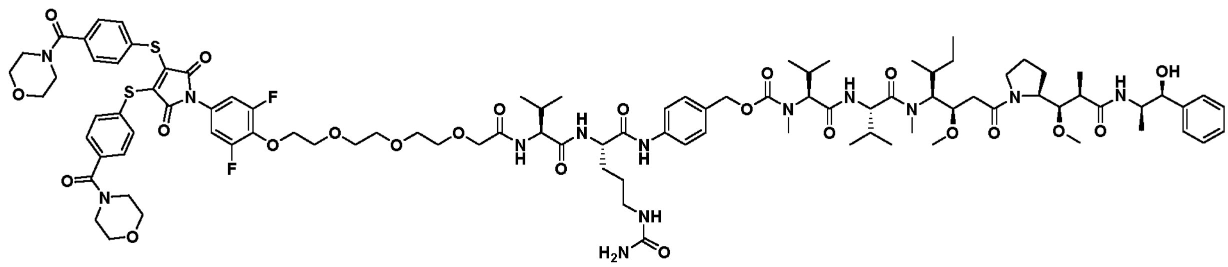

C-3

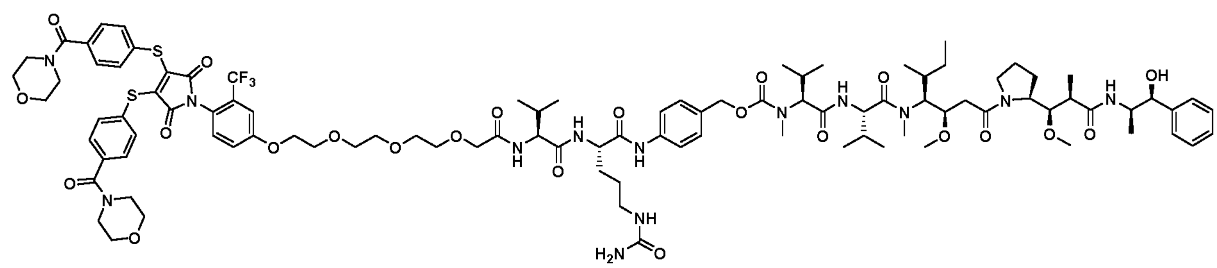

C-4

The compounds were synthesized using the method disclosed in WO2018/095422A1 and all the products were yellow solid. LC-MS (ESI) M+1: 1927 (C-1), 1987 (C-2), 1963 (C-3) and 1995 (C-4), respectively.

Drug-containing linker MC-VC-MMAE was purchased from MCE (MedChemExpress).

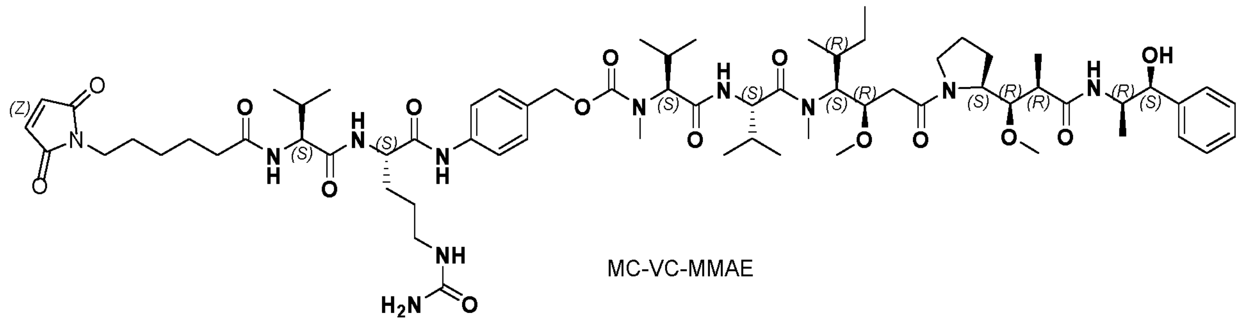

MC-VC-MMAE

**2.2** Preparation of site-specifically conjugated antibody-drug conjugates

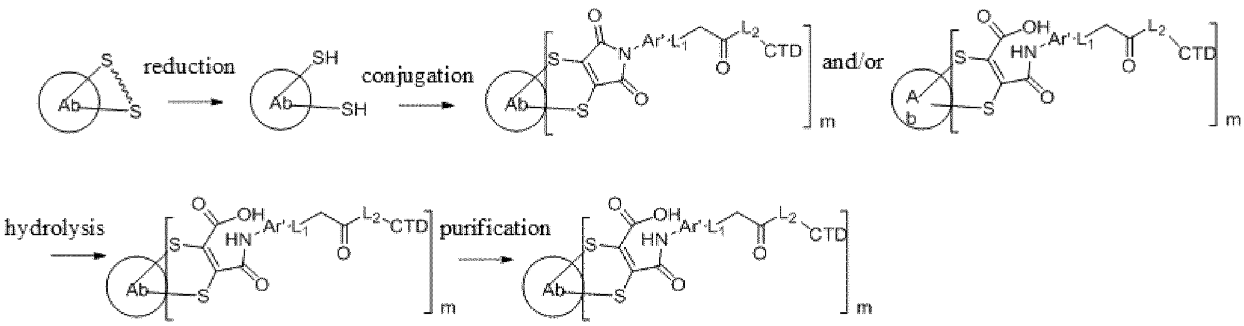


The method for preparing an antibody-drug conjugate was as described in the patent application CN202011046911.X, comprising: reducing an antibody to break disulfide bonds, and conjugating the reduced antibody with a drug-containing linker to form an antibody-drug conjugate; opening the maleimide ring contained in the antibody-drug conjugate via hydrolysis, and purifying the obtained product to provide an antibody-drug conjugate with DAR 4 (the content of the patent application CN202011046911.X is hereby incorporated by reference in its entirety).

An exemplary method used in detail is as follows:

Sample reduction and conjugation: A sample containing an antibody was displaced into a buffer consisting of 50 mM sodium chloride and 50 mM disodium hydrogen phosphate-sodium dihydrogen phosphate, pH 7.4, with a NAP-25 desalting column packed with Sephadex G-25, in which the antibody concentration was diluted to 10 mg/mL. 10 mL of the diluted antibody sample (100 mg in total) was taken, into which an aqueous solution of TCEP (Sigma-Aldrich) at a concentration of 10 mg/mL was added at an equivalent molar ratio of 10: 1 (the reducing agent: the antibody). After incubation for 2 hours, the reaction solution was subjected to buffer exchange with a Sephadex G-25 desalting column, into a buffer consisting of 50 mM NaCl and 50 mM disodium hydrogen phosphate-sodium dihydrogen phosphate, pH 7.0.

The reduced antibody was diluted to 5 mg/mL in the buffer, into which 1.33 mL of N,N-Dimethylacetamide (DMA, as a pre-solvent) which accounted for 7.4% of the total reaction volume and a solution of a drug-containing linker in DMA (10 mg/mL) at an equivalent molar ratio of 5.5: 1 (the drug: the antibody) were added sequentially. The reaction was stirred at room temperature for 60 minutes, and then was subjected to buffer exchange into a buffer consisting of disodium hydrogen phosphate-sodium dihydrogen phosphate, pH 8.0, with a NAP-25 desalting column packed with Sephadex G-25, to remove excessive drug-containing linker. The obtained solution was heated in water bath at 37 °C for 3 hours. Prior to and after the heating in water bath, the solution was sampled for analyzing the structure of the conjugated product contained therein using mass spectrometry. The analysis results of different conjugated products showed that prior to the heating in water bath, the conjugated products were of a structure represented by formula Ia, while a fully ring-opened structure represented by formula Ib was obtained for the conjugated products after the heating in water bath, i.e. they were 100% hydrolyzed.

Sample purification: the solution as described above was concentrated using an AMICOM ultrafiltration centrifuge tube, to a concentration of about 15 mg/mL. A buffer consisting of 50 mM disodium hydrogen phosphate-sodium dihydrogen phosphate and 3 M ammonium phosphate was added into the solution to achieve a conductivity of 74 ms/cm. Then it was applied to a hydrophobic column packed with Butyl-Sepharose 4 FF (purchased from GE Healthcare), using

phase A: a buffer consisting of 50 mM disodium hydrogen phosphate-sodium dihydrogen phosphate and 0.45 M ammonium sulfate; phase B: a buffer consisting of 50 mM disodium hydrogen phosphate-sodium dihydrogen phosphate. 12 column-volume linear gradient elution with phase B from 0% to 80% and isocratic gradient elution with 100% phase B were conducted, and main peaks were collected.

The final sample obtained was subjected to buffer exchange into a buffer consisting of 50 mM disodium hydrogen phosphate-sodium dihydrogen phosphate, pH 7.4 using an AMICOM ultrafiltration centrifuge tube, which was then filtered through a 0.22 μm filter (Sartorius stedim Ministart).

If the actual reaction scale was larger than or equal to 1 g, the NAP-25 desalting column packed with Sephadex G-25 and the AMICOM ultrafiltration centrifuge tube were replaced with HYDRO-30kD (Sartorius) ultrafiltration membrane, with the equivalent ratios used in the reaction unchanged. Other main parameters were unchanged either.

**2.3** Physicochemical characterization of site-specifically conjugated antibody-drug conjugates

**a.** Ultraviolet spectrophotometry used to determine the drug-to-antibody ratio (UV-DAR method) and concentration

The concentration of an antibody-drug conjugate can be obtained by measuring the UV absorbances at 280 nm and the absorption wavelengths characteristic of small molecules and calculating as follows.

**a1.** Determination of the drug-to-antibody ratio (DAR) of randomly conjugated ADCs

It is known from a literature [Clin Cancer Res. 2004 Oct 15;10(20):7063-70] that, DAR (drug-to-antibody ratio) = $(\varepsilon_{280}^{Ab} - A_{280} / A_{248} \times \varepsilon_{248}^{Ab}) / (A_{280} / A_{248} \times \varepsilon_{248}^{D} - \varepsilon_{280}^{D})$, in which $\varepsilon_{280}^{Ab}$ is the molar absorption coefficient of an antibody at 280 nm, $A_{280}$ is the UV absorbance of an antibody-drug conjugate containing the antibody at 280 nm, $A_{248}$ is the UV absorbance of the antibody-drug conjugate at 248 nm which is an absorption wavelength characteristic of the drug-containing linker in the antibody-drug conjugate, $\varepsilon_{248}^{Ab}$ is the molar absorption coefficient of the antibody at 248 nm which is an absorption wavelength characteristic of the drug-containing linker, $\varepsilon_{248}^{D}$ is the molar absorption coefficient of the drug-containing linker at 248 nm, and $\varepsilon_{280}^{D}$ is the molar absorption coefficient of the drug-containing linker at 280 nm, and in which:

| Antibody/small molecule | $\varepsilon_{280}^{Ab}$ | $\varepsilon_{248}^{Ab}$ | $\varepsilon_{248}^{D}$ | $\varepsilon_{280}^{D}$ |
|---|---|---|---|---|
| Enfortumab | 200895 | 72266 | | |
| hH2L1 | 208776 | 78129 | | |
| Hz10 | 203460 | 73069 | | |
| hH1L2mut1 | 203837 | 75632 | | |
| Hz63 | 200869 | 69845 | | |
| mc-vc-MMAE | | | 15900 | 1500 |

**a2.** Determination of the drug-to-antibody ratio (DAR) of site-specifically conjugated ADCs

It is known from the literature [Clin Cancer Res. 2004 Oct 15;10(20):7063-70] that, DAR (drug-to-antibody ratio) = $(\varepsilon_{280}^{Ab} - A_{280} / A_{251} \times \varepsilon_{251}^{Ab}) / (A_{280} / A_{251} \times \varepsilon_{251}^{D} - \varepsilon_{280}^{D})$, in which $\varepsilon_{280}^{Ab}$ is the molar absorption coefficient of an antibody at 280 nm, $A_{280}$ is the UV absorbance of an antibody-drug conjugate containing the antibody at 280 nm, $A_{251}$ is the UV absorbance of the antibody-drug conjugate at 251 nm which is an absorption wavelength characteristic of the drug-containing linker in the antibody-drug conjugate, $\varepsilon_{251}^{Ab}$ is the molar absorption coefficient of the antibody at 251 nm which is an absorption wavelength characteristic of the drug-containing linker, $\varepsilon_{251}^{D}$

is the molar absorption coefficient of the drug-containing linker at 251 nm, and $\varepsilon_{280}^{D}$ is the molar absorption coefficient of the drug-containing linker at 280 nm, and in which:

| Antibody/small molecule | $\varepsilon_{280}^{Ab}$ | $\varepsilon_{251}^{Ab}$ | $\varepsilon_{251}^{D}$ | $\varepsilon_{280}^{D}$ |
|---|---|---|---|---|
| Enfortumab | 200895 | 67251 | | |
| hH2L1 | 208776 | 71247 | | |
| Hz10 | 203460 | 68210 | | |
| hH1L2mut1 | 203837 | 70084 | | |
| Hz63 | 200869 | 65892 | | |
| BL20E | | | 22806 | 8304 |
| note: BL20E is the product obtained by removing the leaving group R from the compound C-3 above and opening the maleimide ring. | | | | |

**a3.** Determination of the concentration of ADCs

Since the total absorbance at a certain wavelength is equal to the sum of the absorbances of all the absorbent chemical species present in the system (additive nature of the absorbance), it is assumed that if the molar absorption coefficients of the antibody and the drug-containing linker contained in an ADC do not change prior to and after the conjugation of the antibody with the drug-containing linker, the concentration of then ADC follows the relationship:

$$A_{280} = \varepsilon_{280}^{ADC} \times C_{ADC} \times L = (\varepsilon_{280}^{D} \times DAR + \varepsilon_{280}^{Ab})\, C_{ADC} \times L.$$

Thus, the molar concentration (mol/L) of the antibody-drug conjugate $C_{ADC} = A_{280} / (\varepsilon_{280}^{Ab} + \varepsilon_{280}^{D} \times DAR)$.

Therefore, the concentration (g/L) of the antibody-drug conjugate $C_{ADC} = A_{280} / (\varepsilon_{280}^{Ab}+\varepsilon_{280}^{D} \times DAR) \times MW_{ADC} = A_{280} / (\varepsilon_{280}^{Ab} + \varepsilon_{280}^{D} \times DAR) \times (MW_{ab} + MW_{D} \times DAR)$, in which $MW_{ADC}$ is the molecular weight of the antibody-drug conjugate, $MW_{ab}$ is the molecular weight of the antibody, and $MW_D$ is the molecular weight of the drug-containing linker; and the protein concentration can be obtained by putting the DAR value into the formula.

**b-1.** Hydrophobic chromatography (HIC-HPLC) used to determine the DAR values of site-specifically conjugated antibody-drug conjugates

Sample preparation: a sample was diluted to 2.0 mg/mL with mobile phase B, and then was centrifuged at 12000 rpm for 10 min; and supernatant obtained was taken for HPLC analysis.
Chromatographic column: Sepax Proteomix HIC Butyl-NP5, 5 μm, 4.6 mm × 35 mm;
Mobile phase A: 0.025 M phosphate +1.2 M ammonium sulphate (pH 7.0);
Mobile phase B: 0.025 M phosphate (pH 7.0);
Mobile phase C: 100% IPA;
Flow rate: 0.8 mL/min;
Detection wavelength: 280 nm;
Column temperature: 30 °C;
Loading volume: 20 μL;
Chromatographic gradient used for HIC analysis:

| Time (min) | Mobile phase A (%) | Mobile phase B (%) | Mobile phase C (%) |
|---|---|---|---|
| 0 | 100 | 0 | 0 |

(continued)

| Time (min) | Mobile phase A (%) | Mobile phase B (%) | Mobile phase C (%) |
|---|---|---|---|
| 1 | 100 | 0 | 0 |
| 12 | 0 | 80 | 20 |
| 16 | 0 | 80 | 20 |
| 17 | 100 | 0 | 0 |
| 21 | 100 | 0 | 0 |

Formula for DAR calculation:

$$DAR = \Sigma\ (\text{weighted peak area}) / 100, \text{ i.e., } DAR = (\text{D0 peak area ratio} \times 0 + \text{D1 peak area ratio} \times 1 + \text{D2 peak area ratio} \times 2 + \text{D3 peak area ratio} \times 3 + \text{D4 peak area ratio} \times 4 + \text{D5 peak area ratio} \times 5 + \text{D6 peak area ratio} \times 6 + \text{D7 peak area ratio} \times 7 + \text{D8 peak area ratio} \times 8) / 100.$$

**b-2.** Hydrophobic chromatography (HIC-HPLC) used to determine the DAR values of randomly conjugated antibody-drug conjugates

Sample preparation: a sample was diluted to 2.0 mg/mL with mobile phase B, and then was centrifuged at 12000 rpm for 10 min; and supernatant obtained was taken for HPLC analysis.
Chromatographic column: TOSOH Butyl NPR, 2.5 μm, 4.6 mm × 100 mm;
Mobile phase A: 125 mM phosphate + 2.5 M ammonium sulphate (pH 6.8);
Mobile phase B: 125 mM phosphate (pH 6.8);
Mobile phase C: 100% IPA;
Mobile phase D: $H_2O$;
Flow rate: 0.7 mL/min;
Detection wavelength: 280 nm;
Column temperature: 30 °C;
Loading volume: 10 μL;
Chromatographic gradient used for HIC analysis:

| Time (min) | Mobile phase A (%) | Mobile phase B (%) | Mobile phase C (%) | Mobile phase D (%) |
|---|---|---|---|---|
| 0 | 50 | 0 | 5 | 45 |
| 10 | 0 | 50 | 5 | 45 |
| 20 | 0 | 50 | 5 | 45 |
| 20.1 | 50 | 0 | 5 | 45 |
| 35 | 50 | 0 | 5 | 45 |

Formula for DAR calculation:

$$DAR = \Sigma\ (\text{weighted peak area}) / 100, \text{ i.e., } DAR = (\text{D0 peak area ratio} \times 0 + \text{D1 peak area ratio} \times 1 + \text{D2 peak area ratio} \times 2 + \text{D3 peak area ratio} \times 3 + \text{D4 peak area ratio} \times 4 + \text{D5 peak area ratio} \times 5 + \text{D6 peak area ratio} \times 6 + \text{D7 peak area ratio} \times 7 + \text{D8 peak area ratio} \times 8) / 100.$$

**c.** Mass spectrometry (LC-MS) used to determine the DAR values

Sample preparation: an appropriate amount of a sample was placed in an ultrafiltration tube, and was subjected to buffer exchange into a buffer consisting of 50 mM NH4HCO3 (pH 7.1). Upon supplementing the buffer, the obtained sample was subjected to ultrafiltration centrifugation (13000 g × 5 min). 8 μL of PNGase F was added into the sample, which was in turn incubated at 37 °C for 5 h for desugarization. After the incubation, the sample was centrifuged at 12000 rpm for 5 min, and supernatant obtained was added into a sample vial as a test sample for later testing.

Chromatographic column: PolyLC ® PolyHYDROXYETHYLA Column, 300 Å, 5um, 2.1 mm × 200 mm;
Mobile phase: 50 mM ammonium acetate, pH 7.0;
Running time: 10 min;
Flow rate: 0.1 mL/min;
Loading volume: 2 μL;
Column temperature: 25 °C;
Detection wavelength: 280 nm;
Ionization mode: ESI positive;
Drying gas temperature: 325 °C;
Drying gas flow rate: 8 L/min;
Atomizer pressure: 20 psig;
Sheath gas temperature: 325 °C;
Sheath gas flow rate: 12 L/min;
Scanning setting: 900-8000 m/z.

**d.** Size exclusion chromatography (SEC-HPLC) used to determine the molecular size heterogeneity of molecules

Sample preparation: a sample was diluted to 1.0 mg/mL with a mobile phase and then was centrifuged at 12000 rpm for 10 min; and supernatant obtained was taken for analysis.

Chromatographic column: TOSOH, TSKgel G3000SW$_{XL}$, 5 μm, 7.8 mm × 300 mm;
Mobile phase: 100 mM phosphate +200 mM arginine hydrochloride, 5% isopropanol (pH 6.8);
Flow rate: 0.6 mL/min;
Detection wavelength: 280 nm;
Column temperature: 30 °C;
Loading volume: 20 uL;
Elution time: 25 min;
Elution gradient: isocratic elution.

**e.** Non-Reducing Capillary Electrophoresis-Sodium Dodecyl Sulfate (NR-CE-SDS) to determine the purity

The determination was conducted according to the method "Determination of molecular size variants of monoclonal antibodies" described in General rule 3127 of Part IV of Chinese Pharmacopoeia.

<u>Example 1</u> Preparation and physicochemical analysis of antibody-drug conjugates targeting Nectin-4

Anti-Nectin-4 antibodies Ref (prepared by reference to the sequences of Enfortumab provided in WHO Drug Information), 42D20-hz63, 42D20-hz10, hH2L1, hH1L2mut1 and irrelevant IgG1 (Bio-Rad Antibodies, Cat No.: MCA928) (each 100 mg) were conjugated with the drug-containing linkers C-1 and C-3 respectively, as described in section **2.2** in the present Group 2 of Examples, and site-specifically conjugated ADCs 1a, 1b, 1c, 1d, 1e, 1f and 2a, 2b, 2c, 2d, 2e were obtained.

Anti-Nectin-4 antibody hH2L1 was conjugated with the drug-containing linker C-3 on a large scale, as described in section **2.2** in the present Group 2 of Examples, using the method for preparing an antibody-drug conjugate described in the patent application CN202011046911.X, and ADC 3d was obtained.

Anti-Nectin-4 antibodies 42D20-hz63, 42D20-hz10, and the IgG1 (each 100 mg) as well as Ref, hH2L1, and hH1L2mut1 (each 500 mg) were conjugated with the drug-containing linker MC-VC-MMAE, using the method described in the patent publication US 2009/0010945 A1, and randomly conjugated ADCs 4a, 4b, 4c, 4d, 4e, and 4f were obtained.

The physicochemical properties of the obtained site-specifically conjugated ADCs and randomly conjugated ADCs were determined as described in sections **2.3 a, 2.3 b-1, 2.3 b-2, 2.3 d as well as 2.3 c,** and **2.3 e** in the present Group 2 of Examples, and the results are provided in Tables 1 to 3 and FIG.1, respectively.

Table 1. Results of quality characterization of the antibody-drug conjugates targeting Nectin-4

| ADC | Antibody | Drug-containing linker | DAR by UV | DAR by HIC | SEC (%) |
|---|---|---|---|---|---|
| 1a | Ref (Enfortumab) | C-1 | 3.7 | 4.0 | 99.1 |
| 1b | 42D20-hz10 | C-1 | 4.2 | 4.0 | 98.7 |
| 1c | 42D20-hz63 | C-1 | 4.1 | 4.0 | 97.9 |

(continued)

| ADC | Antibody | Drug-containing linker | DAR by UV | DAR by HIC | SEC (%) |
|---|---|---|---|---|---|
| 1d | hH2L1 | C-1 | 4.3 | 4.0 | 99.7 |
| 1e | hH1L2mut1 | C-1 | 4.4 | 4.0 | 99.3 |
| 1f | IgG1 | C-1 | 4.3 | 4.0 | 98.7 |
| 2a | Ref (Enfortumab) | C-3 | 3.9 | 4.0 | 99.3 |
| 2b | 42D20-hz10 | C-3 | 3.9 | 4.0 | 99.1 |
| 2c | 42D20-hz63 | C-3 | 4.0 | 4.0 | 98.5 |
| 2d | hH2L1 | C-3 | 4.0 | 4.0 | 99.4 |
| 2e | hH1L2mut1 | C-3 | 3.9 | 4.0 | 98.8 |
| 2f | IgG1 | C-3 | 3.7 | 4.0 | 97.9 |
| 3d | hH2L1 | C-3 | 4.0 | 4.0 | 98.7 |

Table 2. Results of quality characterization of the control antibody-drug conjugates targeting Nectin-4

| ADC | Antibody | Drug-containing linker | DAR by UV | DAR by HIC | SEC (%) |
|---|---|---|---|---|---|
| 4a | Ref (Enfortumab) | MC-VC-MMAE | 4.2 | 4.0 | 98.2 |
| 4b | 42D20-hz10 | MC-VC-MMAE | 4.0 | 3.7 | 96.9 |
| 4c | 42D20-hz63 | MC-VC-MMAE | 4.2 | 3.8 | 94.1 |
| 4d | hH2L1 | MC-VC-MMAE | 4.1 | 4.2 | 98.5 |
| 4e | hH1L2mut1 | MC-VC-MMAE | 4.1 | 4.3 | 99.3 |
| 4f | IgG1 | MC-VC-MMAE | 4.2 | 4.2 | 99.5 |

Table 3. Results of mass spectrometric characterization prior to and after hydrolysis

| ADC | Measured molecular weight | Compounds in the system |
|---|---|---|
| 3d, prior to hydrolysis | 155015 | Mixed compounds of formula Ia and formula Ib in the system |
| 3d, after hydrolysis | 155034 | Compound of formula Ib in the system |

The measured molecular weights by Mass Spectrometry showed that the sample prior to hydrolysis contained a mixture of compounds having the structures represented by the formulas Ia and Ib, while the sample after hydrolysis contained compound having the structure represented by formula Ib only, which was obtained by fully opening the maleimide ring.

**Group 3 of Examples** Study of in vitro killing effect and in vivo efficacy of antibody-drug conjugates

The following methods are used for illustrative experiments::

**3.1** The method for studying in vitro killing effect

BT474 cells, i.e. human breast ductal carcinoma cells (purchased from ATCC) were utilized in this experiment. The density of BT474 cells was adjusted to $1.5\times10^4$/mL in complete medium (prepared by mixing 45 mL of RPMI 1640 medium and 5 mL of FBS). The cells were added at 100 μL/well into cell culture plates and cultured overnight. Next day, ADC samples were diluted to 50 μg/mL with the complete medium as above, followed by 4-fold gradient dilution to obtain a total of 9 gradients plus zero concentration. All the samples were prepared for 3 replicate wells. Negative control (cell + culture medium) and blank control (no cell, culture medium only) were set. The diluted ADC samples were added to the overnight-cultured cell culture plates, at 100 μL/well. Then the cells plates were incubated in a cell incubator

for 96 h. The cell culture plates were taken out and MTS was added into the plates at 40 μL/well, which were then incubated in an incubator at 37 °C for 2-4 h. The cell plates were taken out and OD values at 490 nm were read.

**3.2** The method for studying in vivo efficacy

BT474 cells, i.e. human breast ductal carcinoma cells (purchased from ATCC) and MDA-MB-468 cells, i.e. human breast carcinoma cells (purchased from ATCC) were utilized to inoculate Nude mice (BALB/cJGpt-Foxn 1nu/Gpt, 4-5 weeks old) for establishing mouse models, respectively. When the tumor grew to 100-200 $mm^3$, the mice were intravenously injected (IV) in a volume of 10 mL/kg, respectively; and the mice of the vehicle group were intravenously injected the same volume of vehicle (physiological saline). Dosages and administration schemes in details are set forth below. Tumor volumes were measured 2 times per week, and the mice were weighed and the data was recorded.

When the experiment was finished, the experimental endpoint was reached, or if the tumor volume reached 1500 mm3, the animals were sacrificed under $CO_2$ anesthesia and the tumors were dissected and photographed.

**3.3** The method for evaluating in vivo efficacy

This experiment is to examine the influence of the ADCs on tumor growth, and specific index T/C (%) or Tumor Growth Inhibition (TGI (%)) was used.

Tumor diameters were measured twice per week with a vernier caliper and tumor volume (V) was calculated as follows: $V = 1/2 \times a \times b^2$, in which a and b is length and width, respectively.

$T/C (\%) = (T - T_0) / (C - C_0) \times 100$, in which T and C are the tumor volumes at the end of the experiment; and $T_0$ and $C_0$ are the tumor volumes at the beginning of the experiment.

$$\text{Tumor Growth Inhibition (TGI) (\%)} = 100 - T/C\ (\%).$$

When tumor regression occurred, Tumor Growth Inhibition (TGI) (%) = $100 - (T - T_0) / T_0 \times 100$.

Partial tumor regression (PR) was recorded if the tumor shrunk from its initial volume, i.e., $T < T_0$ or $C < C_0$; and complete tumor regression (CR) was recorded if the tumor completely disappeared.

Example 1 Study of in vitro killing effect of antibody-drug conjugates targeting Nectin-4

ADCs produced by conjugating different drug-containing linkers with different anti-Nectin-4 antibodies were grouped and studied as described in section **3.1** in the present Group 3 of Examples. The founding was as follows.

When different antibodies were conjugated with MC-VC-MMAE to form ADCs, the ADC in which antibody hH2L1 was conjugated exhibited a level of in vitro killing effect which was comparable to that of the ADC in which the control antibody Ref (Enfortumab) was conjugated, while slightly better than those of the ADCs in which the antibodies 42D20-hz10, 42D20-hz63 and hH1L2mut1 were conjugated. Further, the comparison between ADCs 2a, 1a, and 2e showed, compounds C-3 and C-1 had comparable in vivo activities. The results are shown in Table 4.

Table 4. Study of in vitro killing effect of different antibodies and drug-containing linkers

| Plate # | ADC | Antibody | Drug-containing linker | EC50 (ng/ml) | Maximum cell killing percentage (%) |
|---|---|---|---|---|---|
| Plate 1 | 4b | 42D20-hz10 | MC-VC-MMAE | 16.34 | 55 |
| | 4a | Ref (Enfortumab) | MC-VC-MMAE | 10.89 | 62.4 |
| | 4d | hH2L1 | MC-VC-MMAE | 11.24 | 55 |
| Plate 2 | 4e | hH1L2mut1 | MC-VC-MMAE | 132.5 | 30.7 |
| | 4a | Ref (Enfortumab) | MC-VC-MMAE | 17.2 | 60.7 |
| | 4d | hH2L1 | MC-VC-MMAE | 13.79 | 55.7 |
| Plate 3 | 4a | Ref (Enfortumab) | MC-VC-MMAE | 11.74 | 47.6 |
| | 4e | hH1L2mut1 | MC-VC-MMAE | 138.1 | 27.2 |
| | 4b | 42D20-hz10 | MC-VC-MMAE | 12.060 | 41.0 |

(continued)

| Plate # | ADC | Antibody | Drug-containing linker | EC50 (ng/ml) | Maximum cell killing percentage (%) |
|---|---|---|---|---|---|
| Plate 4 | 4a | Ref (Enfortumab) | MC-VC-MMAE | 14.13 | 58.5 |
| | 4b | 42D20-hz10 | MC-VC-MMAE | 14.04 | 49.6 |
| | 4c | 42D20-hz63 | MC-VC-MMAE | 11.34 | 45.8 |
| Plate 5 | 2a | Ref (Enfortumab) | C-3 | 12.42 | 39.9 |
| | 1a | Ref (Enfortumab) | C-1 | 8.345 | 37.8 |
| | 2e | hH1L2mut1 | C-3 | N/A | N/A |

Example 2 Study of in vivo efficacy of antibody-drug conjugates targeting Nectin-4 (1)

A mouse model was established using BT474 cells and ADCs produced by conjugating different drug-containing linkers with different anti-Nectin-4 antibodies were grouped and studied, as described in sections **3.2** and **3.3** in the present Group 3 of Examples.

Table 5. Grouping and administration schemes of different ADCs

| Administration group | Antibody | Drug-containing linker | Dosage (mg/kg) | Administration route | Administration timepoint | Administration volume (mL/kg) |
|---|---|---|---|---|---|---|
| Vehicle | None | None | None | IV | D0 | 10 |
| 4a | Ref (Enfortumab) | MC-VC-MMAE | 5 | IV | D0 | 10 |
| 4c | 42D20-hz63 | MC-VC-MMAE | 5 | IV | D0 | 10 |
| 2c | 42D20-hz63 | C-3 | 5 | IV | D0 | 10 |
| 4b | 42D20-hz10 | MC-VC-MMAE | 5 | IV | D0 | 10 |
| 2b | 42D20-hz10 | C-3 | 5 | IV | D0 | 10 |
| 4f | IgG1 | MC-VC-MMAE | 5 | IV | D0 | 10 |
| 2f | hH1L2mut1 | MC-VC-MMAE | 5 | IV | D0 | 10 |

The results are shown in FIG. 2 and Table 6.

Table 6. Results of in vivo efficacy of different ADCs

| Group | Average tumor volume (mm3) | | | Average tumor volume (mm3) | | | T/C (%) | TGI (%) | P value | Number of animals per group at the beginning of the experiment | Number of animals per group at the end of the experiment |
|---|---|---|---|---|---|---|---|---|---|---|---|
| | D0 | | SEM | D26 | | SEM | D26 | D26 | D26 | | |
| Vehicle | 118.1 | ± | 2.7 | 2005.1 | ± | 253.9 | - | - | - | 10 | 10 |
| 4a, 5mg/kg | 115.6 | ± | 4.0 | 169.7 | ± | 50 | 3 | 97 | 0.000 | 6 | 6 |
| 4c, 5mg/kg | 112.0 | ± | 3.6 | 421.3 | ± | 156.7 | 16 | 84 | 0.001 | 6 | 6 |
| 2c, 5mg/kg | 113.0 | ± | 3.5 | 220.9 | ± | 59.3 | 6 | 94 | 0.000 | 6 | 6 |
| 4b, 5mg/kg | 114.8 | ± | 4.3 | 289.3 | ± | 47.3 | 9 | 91 | 0.000 | 6 | 6 |
| 2b, 5mg/kg | 115,1 | ± | 3.6 | 160.5 | ± | 28.6 | 2 | 98 | 0.000 | 6 | 6 |
| 4f, 5mg/kg | 114.3 | ± | 3.6 | 1879.0 | ± | 347.4 | 94 | 6 | 0.777 | 6 | 6 |
| 2f, 5mg/kg | 114.8 | ± | 4.1 | 1230.1 | ± | 219.4 | 59 | 41 | 0.058 | 6 | 6 |

note: the P values in the Table are determined by comparing with the vehicle group.

Example 3 Study of in vivo efficacy of antibody-drug conjugates targeting Nectin-4 (2)

A mouse model was established using MDA-MB-468 cells and ADCs produced by conjugating different drug-containing linkers with different anti-Nectin-4 antibodies were grouped and studied, as described in sections **3.2** and **3.3** in the present Group 3 of Examples.

Table 7. Grouping and administration schemes of different ADCs

| Administration group | Antibody | Drug-containing linker | Dosage (mg/kg) | Administration route | Administration timepoint | Administration volume (mL/kg) |
|---|---|---|---|---|---|---|
| Vehicle | None | None | None | IV | D0 | 10 |
| 4d | hH2L1 | MC-VC-MMAE | 5 | IV | D0 | 10 |
| 2d | hH2L1 | C-3 | 5 | IV | D0 | 10 |
| 4a | Ref (Enfortumab) | MC-VC-MMAE | 5 | IV | D0 | 10 |

The results are shown in FIG. 3 and Table 8.

Table 8. Results of in vivo efficacy of different ADCs

| Group | TV(DO) | SEM | TV(D24) | SEM | T/C(%) (D24) | TGI% (D24) | P value | PR (D24) | Number of animals (D0) | Number of animals (D24) |
|---|---|---|---|---|---|---|---|---|---|---|
| Vehicle | 112.9 | ± 2.5 | 589.6 | ± 68.3 | - | - | - | 0 | 10 | 10 |
| 4d, 5 mg/kg | 116.1 | ± 3.0 | 333.0 | ± 35.0 | 46 | 54 | 0.015 | 0 | 6 | 6 |
| 2d, 5 mg/kg | 112.0 | ± 3.9 | 110.6 | ± 10.8 | -1 | 101 | 0.000 | 4 | 6 | 6 |
| 4a, 5 mg/kg | 115.7 | ± 2.7 | 248.7 | ± 26.5 | 28 | 72 | 0.002 | 0 | 6 | 6 |

note: the P values in the Table are determined by comparing with the vehicle group. TV: tumor volume, $mm^3$.

Group 4 of Examples Evaluation of efficacy of antibody-drug conjugate 3d as a candidate drug in different tumor models

The following methods are used for illustrative experiments:

**4.1** Grouping and experiment design

MDA-MB-468 cells, i.e. human breast carcinoma cells (purchased from ATCC), NCI-H322 cells, i.e. lung cancer cells (purchased from ATCC), and T24/nectin-4, i.e. bladder cancer cells (available from the cell bank of the Chinese Academy of Science) were utilized respectively to inoculate Nude mice (BALB/cJGpt-Foxn1nu/Gpt, 4-5 weeks old) for establishing mouse models.

The antibody-drug conjugate 3d and the control drug PADCVE (Enfortumab Vedotin, purchased from Seattle Gentics) were grouped and studied (as shown in Table 9 below). The mice were intravenously injected (IV) in a volume of 10 mL/kg, respectively; and the mice of the vehicle group were intravenously injected the same volume of vehicle (physiological saline). Dosages and administration schemes in details are shown in Table 9. Tumor volumes were measured 2 times per week, and the mice were weighed and the data was recorded.

When the experiment was finished, the experimental endpoint was reached, or if the tumor volume reached 1500 $mm^3$, the animals were sacrificed under $CO_2$ anesthesia and the tumors were dissected and photographed.

Table 9. Grouping and administration schemes of different ADCs

| Administration group | Antibody | Drug-containing linker | Dosage (mg/kg) | Administration route | Administration timepoint | Administration volume (mL/kg) |
|---|---|---|---|---|---|---|
| Vehicle | None | None | None | IV | D0 | 10 |
| 3d | hH2L1 | C-3 | 1 | IV | D0 | 10 |
| 3d | hH2L1 | C-3 | 3 | IV | D0 | 10 |
| 3d | hH2L1 | C-3 | 10 | IV | D0 | 10 |
| PADCVE | Ref (Enfortumab) | MC-VC-MMAE | 3 | IV | D0 | 10 |
| PADCVE | Ref (Enfortumab) | MC-VC-MMAE | 10 | IV | D0 | 10 |

**4.2** The method for evaluating in vivo efficacy

The method for evaluating in vivo efficacy was the same as described in Section **3.3** in the Group 3 of Examples.

Example 1 Study of in vivo efficacy of antibody-drug conjugates targeting Nectin-4 in a breast cancer animal model

A mouse model was established using MDA-MB-468 cells and ADCs 3d and PADCVE were grouped and studied, as described in sections **4.1** and **4.2** in the present Group 4 of Examples. The results are shown in FIG. 4 and Table 10.

Table 10. Results of in vivo efficacy of different ADCs

| Group | TV(DO) | | SEM | TV(D21) | | SEM | T/C (%) (D21) | TGI (%) (D21) | P val ue | PR (D21) | Number of animals (D0) | Number of animals (D21) |
|---|---|---|---|---|---|---|---|---|---|---|---|---|
| Vehicle | 132.4 | ± | 3.6 | 739.1 | ± | 20.7 | - | - | - | 0 | 10 | 10 |
| 3d, 1 mg/kg | 133.7 | ± | 3.1 | 628.1 | ± | 60.8 | 82 | 18 | 0.7 21 | 0 | 8 | 8 |
| 3d, 3 mg/kg | 134.6 | ± | 4.6 | 355.5 | ± | 59.7 | 36 | 64 | 0.0 00 | 0 | 8 | 8 |
| 3d, 10 mg/kg | 133.9 | ± | 3.4 | 119.5 | ± | 31.2 | -11 | 111 | 0.0 00 | 7 | 8 | 8 |
| PADCEV®, 3 mg/kg | 133.1 | ± | 3.4 | 516.4 | ± | 33.7 | 63 | 37 | 0.0 00 | 0 | 8 | 8 |
| PADCEV®, | 139.5 | ± | 3.3 | 309.5 | ± | 49.7 | 28 | 72 | 0.0 | 0 | 8 | 8 |
| 10 mg/kg | | | | | | | | 00 | | | | |

Note: the P values in the Table are determined by comparing with the vehicle group. TV: tumor volume, $mm^3$. PR: Partial tumor regression.

Example 2 Study of in vivo efficacy of antibody-drug conjugates targeting Nectin-4 in a lung cancer animal model

A mouse model was established using NCI-H322 cells and ADCs 3d and PADCVE were grouped and studied, as described in sections **4.1** and **4.2** in the present Group 4 of Examples. The results are shown in FIG. 5 and Table 11.

Table 11. Results of in vivo efficacy of different ADCs

| Group | TV(D0) | | SEM | TV(D21) | | SEM | T/C (%) (D21) | TGI (%) (D21) | P value | PR (D21) | Number of animals (D0) | Number of animals (D21) |
|---|---|---|---|---|---|---|---|---|---|---|---|---|
| Vehicle | 121.0 | ± | 1.6 | 676.2 | ± | 41.3 | - | - | - | 0 | 12 | 12 |
| 3d, 1 mg/kg | 122.3 | ± | 1.9 | 325.8 | ± | 36.6 | 37 | 63 | <0.001 | 0 | 8 | 8 |
| 3d, 3 mg/kg | 124.4 | ± | 2.1 | 216.7 | ± | 31.1 | 17 | 83 | <0.001 | 1 | 8 | 8 |
| 3d, 10 mg/kg | 123.1 | ± | 2.0 | 81.1 | ± | 2.5 | -34 | 134 | <0.001 | 8 | 8 | 8 |
| PADCEV®, 3 mg/kg | 125.1 | ± | 1.9 | 325.9 | ± | 33.3 | 36 | 64 | <0.001 | 0 | 8 | 8 |
| PADCEV®, 10 mg/kg | 124.1 | ± | 2.6 | 95.9 | ± | 5.2 | -23 | 123 | <0.001 | 7 | 8 | 8 |
| Note: the P values in the Table are determined by comparing with the vehicle group. TV: tumor volume, $mm^3$. PR: Partial tumor regression. | | | | | | | | | | | | |

Example 3 Study of in vivo efficacy of antibody-drug conjugates targeting Nectin-4 in a bladder cancer animal model

A mouse model was established using T24/nectin-4 cells and ADCs 3d and PADCVE were grouped and studied, as described in sections **4.1** and **4.2** in the present Group 4 of Examples. The results are shown in FIG. 6 and Table 12.

Table 12. Results of in vivo efficacy of different ADCs

| Group | Average weight (g) | | Number of mice | | Average tumor weight (g) (D22) | | SD | TGI (%) (D22) | P value |
|---|---|---|---|---|---|---|---|---|---|
| | at the beginning | at the end | at the beginning | at the end | | | | | |
| Vehicle | 22.1 | 19.5 | 10 | 10 | 2.46 | ± | 0.25 | _ | |
| 3d, 1 mg/kg | 22.0 | 18.8 | 8 | 8 | 1.98 | ± | 0.35 | 19.3 | 0.004 |
| 3d, 3 mg/kg | 22.0 | 22.2 | 8 | 8 | 0.58 | ± | 0.88 | 76.2 | 0.000 |
| 3d, 10 mg/kg | 22.2 | 23.5 | 8 | 8 | 0.08 | ± | 0.03 | 96.9 | 0.000 |
| PADCEV®, 3 mg/kg | 22.0 | 23.0 | 8 | 8 | 0.23 | ± | 0.15 | 90.7 | 0.000 |
| PADCEV®, 10 mg/kg | 22.0 | 23.3 | 8 | 8 | 0.09 | ± | 0.02 | 96.3 | 0.000 |
| Note: the P values in the Table are determined by comparing with the vehicle group. | | | | | | | | | |

**Group 5 of Examples** Evaluation of functions of antibody-drug conjugates

Example 1 Analysis of affinity of the Fab regions of antibody-drug conjugates targeting Nectin-4 A BIAcore assay was used to determine the affinities of antibody-drug conjugates 3d and Padcev for the antigen, i.e., recombinant extra-cellular domain of human Nectin-4 protein.

Human Nectin-4 protein (purchased from Novoprotein Scientific Inc.) was subjected to buffer exchange with a desalting column using running reagent 1 (10 mM N-(2-hydroxyethyl)piperazine-N-2 sulfonic acid, 150 mM sodium chloride, 3 mM ethylene diamine tetraacetic acid, 0.005% Tween-20, with pH adjusted to 7.4). Samples to be detected were diluted to 5 μg/mL with the running reagent 1, and sequentially injected to the experimental channels with His capture chips at a flow rate of 10 μg/min for about 400 RU. The capture of the ligand was not required for the reference channel. The human Nectin-4 protein was diluted to 50, 25, 12.5, 6.25, 3.125, 1.563, 0.781, 0.391, and 0 nM with the corresponding running reagent, and then the diluted human Nectin-4 protein was injected into the experimental channels and the reference channel at a flow rate of 30 μL/min. The association and dissociation time were 120 sec and 300 sec, respectively. The Biocore 8K analysis software was used to calculate KD values for each antibody. The reference channel was used for background subtraction.

The detection results of the affinities of hH2L1, 3d and Padcev for human Nectin-4 are shown in Table 13. The results showed that the affinities (KD) of hH2L1, 3d and Padcev for human Nectin-4 were all at a nanomolar scale, with good batch-to-batch consistency; the affinity of hH2L1 was basically consistent with that of ADC 3d, indicating that the conjugation process for preparing the ADC did not significantly influence the binding of the antibody to Nectin-4; and, Padcev had both association rate constant (ka) and dissociation rate constant (kd) higher than ADC 3d, so accordingly ADC 3d showed a better affinity as a whole than Padcev, about 2 times higher than that of Padcev.

Table 13. Detection results of affinities for the antigen

| Group | ka (1/Ms) | kd (1/s) | KD (nM) |
|---|---|---|---|
| hH2L1 | 5.62E+05 | 1.45E-03 | 2.58 |
| 3d | 5.89E+05 | 1.42E-03 | 2.41 |
| PADCEV | 1.05E+06 | 5.12E-03 | 4.87 |

Example 2 Analysis of the Fc regions of antibody-drug conjugates targeting Nectin-4

A BIAcore assay was used to determine the affinities of antibody hH2L1, and antibody-drug conjugates 3d and Padcev for Fc receptors including: FcRn, human FcγR I (CD64), human FcγR IIa (human CD32a (H167)), human FcγR

IIb (human CD32b), and human FcγR IIIa (human CD16a (V176)). Human FcγR I (CD64), FcγR IIa (CD32a (H167)), FcγR IIb (CD32b), and FcγR IIIa (CD16a (V176)) proteins were diluted to 0.25 μg/mL with running reagent 1 (10 mM N-(2-hydroxyethyl)piperazine-N-2 sulfonic acid, 150 mM sodium chloride, 3 mM ethylene diamine tetraacetic acid, 0.005% Tween-20, with pH adjusted to 7.4), and FcRn protein was diluted to 0.25 μg/mL with running reagent 2 (2 mM sodium dihydrogen phosphate, 10 mM disodium hydrogen phosphate, 137 mM sodium chloride, 2.7 mM potassium chloride, 0.05% tween-20, with pH adjusted to 6.0) and all the diluted proteins were sequentially injected to the experimental channels with His capture chips (FC2) at a flow rate of 10 μg/min for about 40 RU. The capture of the ligands was not required for the reference channel (FC1). Samples to be detected were diluted with corresponding running reagents, and then were injected into the experimental channels and the reference channel at a flow rate of 30 μL/min. Association and dissociation was monitored for certain time periods. The Biocore 8K analysis software was used to calculate KD values for each antibody. The reference channel (FC1) was used for background subtraction.

The detection results of the affinities of hH2L1, 3d and Padcev for Fc receptors are shown in Table 14. The results showed that the affinities of hH2L1, and four batches of 3d for the Fc receptors exhibited no batch-to-batch significant difference; the affinities of ADC 3d and one batch of Padcev for FcγR IIIa were slightly inferior to that of hH2L1; and there was no obvious difference in the affinities of the three for each of the other receptors.

Table 14. Detection results of affinities for the Fc receptors

| Group | Affinity (KD) | | | | |
|---|---|---|---|---|---|
| | FcRn (μM) | FcγRI (nM) | FcγRIIa (μM) | FcγRIIb (μM) | FcγRIIIa (μM) |
| hH2L1 | 0.549 | 13.9 | 4.00 | 35.8 | 0.178 |
| 3d | 0.557 | 9.81 | 3.51 | 13.8 | 0.300 |
| PADCEV | 0.513 | 12.3 | 2.77 | 16.3 | 0.252 |

Example 3 Study of internalization activities of antibody-drug conjugates targeting Nectin-4

A stably transfected cell strain PC-3-Nectin4(2-4) (self-made) highly expressing Nectin-4 was used as a target cell strain to detect the internalization activities of hH2L1, 3d and Padcev.

Experiment method was as follows: the target cells at the logarithmic stage of growth were harvested by centrifugation, adjusted to a density of $5\times10^5$ cells/mL in complete medium (F-12K medium containing 10% FBS and 250 μg/ml G418). 1 mL of the cell suspension obtained was pipetted into EP tubes, i.e., $5\times10^5$ cells in each, which were then centrifuged. The supernatant was discarded and the cells were washed twice with PBS. 200 μL of each of hH2L1, 3d and Padcev samples was added to the cells, which were then placed at 4 °C (as the control group) and 37 °C (as the experiment groups) respectively. After incubation for 2 h, a fluorescently labeled secondary antibody, goat anti human IgG Fc-FITC (purchased from Sigma) was added and incubated for 1 h at 4 °C. After the incubation, the fluorescence intensity was measured with a flow cytometer, and the internalization efficiency of each of the samples was calculated by the formula as follows: percent internalization at the time point tx = (1 - the fluorescence intensity of the sample at 37 °C / the fluorescence intensity of the sample at 4 °C) × 100% (tx is the incubation time period x of the sample with the cells).

The result showed that a relatively high level of the target protein Nectin-4 was expressed on the surface of the PC-3-Nectin4(2-4) cells, to which all the ADC of interest, the naked antibody and Padcev exhibited a rather strong binding capability to Nectin-4 as compared with a irrelevant antibody. From the results obtained, there was no significant difference between the internalization activities of ADC 3d and three batches of stock solutions of the ADC and the internalization activity of Padcev.

The results are shown in Table 15.

Table 15. Detection results of internalization activities of hH2L1, 3d and Padcev

| Group | Average | | | Median | | |
|---|---|---|---|---|---|---|
| | 4 °C | 37 °C | Internalization efficiency | 4 °C | 37 °C | Internalization efficiency |
| Blank | 229 | N/A | N/A | 216 | N/A | N/A |
| hH2L1 | 83591 | 29646 | 64.53% | 78157 | 19948 | 74.48% |
| 3d | 90122 | 42886 | 52.41% | 85562 | 30891 | 63.90% |
| PADCEV | 83966 | 33129 | 60.54% | 78925 | 25038 | 62.28% |

The above description of the embodiments of the present disclosure is not intended to limit the present disclosure, and those skilled in the art may make various changes and modifications to the present disclosure without departing from the spirit of the present disclosure, which should fall within the scope of the appended claims.

## Claims

1. An antibody-drug conjugate targeting Nectin-4 or salt thereof, comprising an anti-Nectin-4 antibody or fragment thereof covalently linked to a drug;
wherein the anti-Nectin-4 antibody or fragment thereof comprises a heavy chain and a light chain comprising heavy chain complementarity determining regions 1 to 3 (CDR-H1, CDR-H2 and CDR-H3) and light chain complementarity determining regions 1 to 3 (CDR-L1, CDR-L2 and CDR-L3) respectively as follows :

   (i) CDR-H1, CDR-H2 and CDR-H3 having amino acid sequences as shown in SEQ ID NO: 11, SEQ ID NO: 12 and SEQ ID NO: 13 respectively; and, CDR-L1, CDR-L2 and CDR-L3 having amino acid sequences as shown in SEQ ID NO: 14, SEQ ID NO: 15 and SEQ ID NO: 16 respectively;
   (ii) CDR-H1, CDR-H2 and CDR-H3 having amino acid sequences as shown in SEQ ID NO: 11, SEQ ID NO: 17 and SEQ ID NO: 13 respectively; and, CDR-L1, CDR-L2 and CDR-L3 having amino acid sequences as shown in SEQ ID NO: 18, SEQ ID NO: 15 and SEQ ID NO: 16 respectively; or
   (iii) CDR-H1, CDR-H2 and CDR-H3 having amino acid sequences as shown in SEQ ID NO: 19, SEQ ID NO: 20 and SEQ ID NO: 13 respectively; and, CDR-L1, CDR-L2 and CDR-L3 having amino acid sequences as shown in SEQ ID NO: 14, SEQ ID NO: 15 and SEQ ID NO: 16 respectively.

2. The antibody-drug conjugate or salt thereof according to claim 1, wherein the antibody-drug conjugate or salt thereof has a formula as follows: Ab-[L-CTD]m, in which Ab represents the anti-Nectin-4 antibody or fragment thereof, L represents a linker, CTD represents the drug, and m represents the average number of the drug coupled per one Ab molecule;

   preferably, CTD is a cytotoxic drug; preferably, CTD is one or more selected from the group consisting of: microtubule inhibitors MMAE, DM1, DM4, Tublysin, amanitin, calicheamicin, Eribulin and derivatives thereof; topoisomerase inhibitors SN38, Exatecan and derivatives thereof; and DNA binding agents PBD, doxorubicin and derivatives thereof;
   preferably, m is 1.0 to 5.0, preferably 3.0 to 4.2, more preferably 3.5 to 4.5, still more preferably 3.8 to 4.2, yet more preferably 3.9 to 4.1, and particularly preferably 4.0.

3. The antibody-drug conjugate or salt thereof according to claim 1 or 2, wherein the heavy chain and the light chain comprised in the anti-Nectin-4 antibody or fragment thereof comprise a heavy chain variable region (VH) and a light chain variable region (VL) respectively, wherein the heavy chain variable region (VH) comprises an amino acid sequence as shown in SEQ ID NO: 1, SEQ ID NO: 3, SEQ ID NO: 5 or SEQ ID NO: 7 or a variant thereof, and the light chain variable region (VL) comprises an amino acid sequence as shown in SEQ ID NO: 2, SEQ ID NO: 4, SEQ ID NO: 6 or SEQ ID NO: 8 or a variant thereof;
preferably, the heavy chain variable region (VH) and the light chain variable region (VL) comprised in the anti-Nectin-4 antibody or fragment thereof comprise respectively:

   (i) the amino acid sequence as shown in SEQ ID NO: 1 or a variant thereof; and, the amino acid sequence as shown in SEQ ID NO: 2 or a variant thereof;
   (ii) the amino acid sequence as shown in SEQ ID NO: 3 or a variant thereof; and, the amino acid sequence as shown in SEQ ID NO: 4 or a variant thereof;
   (iii) the amino acid sequence as shown in SEQ ID NO: 5 or a variant thereof; and, the amino acid sequence as shown in SEQ ID NO: 6 or a variant thereof; or
   (iv) the amino acid sequence as shown in SEQ ID NO: 7 or a variant thereof; and, the amino acid sequence as shown in SEQ ID NO: 8 or a variant thereof.

4. The antibody-drug conjugate or salt thereof according to any one of claims 1 to 3, wherein the anti-Nectin-4 antibody or fragment thereof is in any form, e.g., a monoclonal antibody, a single chain antibody, a diabody, a single domain antibody, a nanobody, a fully or partially humanized antibody, or a chimeric antibody and the like against Nectin-4; alternatively, the antibody or fragment thereof is a half-antibody or an antigen-binding fragment of the half-antibody against Nectin-4, e.g., scFv, BsFv, dsFv, $(dsFv)_2$, Fab, Fab', $F(ab')_2$, or Fv;

preferably, the Nectin-4 is mammal Nectin-4, preferably primate Nectin-4, more preferably human Nectin-4.

**5.** The antibody-drug conjugate or salt thereof according to any one of claims 1 to 4, wherein the antibody is a monoclonal antibody, preferably a murine, chimeric, or humanized monoclonal antibody; more preferably, the heavy chain constant region of the monoclonal antibody is of IgG1 or IgG4 subtype and the light chain constant region of the monoclonal antibody is of kappa type; alternatively, the antibody is an immunoglobulin, in particular IgA, IgD, IgE, IgG or IgM, e.g., a human subtype of IgA, IgD, IgE, IgG or IgM, more preferably a human IgG1, IgG2, IgG3 or IgG4 subtype;
preferably, the anti-Nectin-4 antibody or fragment thereof comprises a heavy chain constant region comprising the amino acid sequence as shown in SEQ ID NO: 9 or a variant thereof; alternatively, the anti-Nectin-4 antibody or fragment thereof comprises a light chain constant region comprising the amino acid sequence as shown in SEQ ID NO: 10 or a variant thereof.

**6.** The antibody-drug conjugate or salt thereof according to any one of claims 1 to 5, wherein the antibody-drug conjugate or salt thereof has a structure represented by the formula Ia and/or Ib as follows:

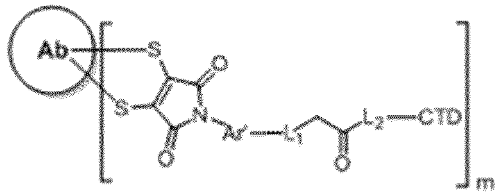

Ia

and/or

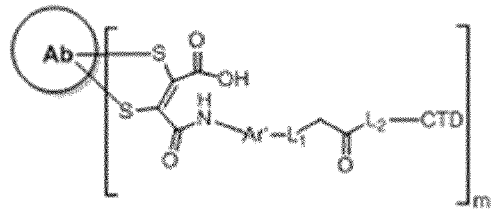

Ib

wherein:

Ab is the anti-Nectin-4 antibody or fragment thereof;
Ar' is any one selected from the group consisting of: substituted or unsubstituted C6-C10 arylene and substituted or unsubstituted 5-12 membered heteroarylene, wherein the substitution refers to the replacement of a hydrogen atom on a group by one or more substituents selected from the group consisting of: halogen (F, Cl, Br or I), halogenated alkyl (e.g. halogenated C1-C6 alkyl, preferably halogenated C1-C4 alkyl, e.g. trifluoromethyl) and alkoxy (e.g. C1-C6 alkoxy, preferably C1-C4 alkoxy, e.g. methoxy);
$L_1$ is -O(CH2CH2O)n- linked to Ar', wherein n is an integer in the range from 1 to 24, preferably from 1 to 10, more preferably from 3 to 5;
$L_2$ is an enzyme cleavable fragment, e.g., a dipeptide or a tripeptide or a tetrapeptide or a combination thereof with a cleavable self-immolating linker (i.e., a polypeptide fragment consisting of 2-4 amino acids or a combination of the polypeptide fragment with a cleavable self-immolating linker), such as Val-Ala, Val-Ala-PAB, Val-Cit, Val-Cit-PAB, Phe-Lys-PAB, Ala-Ala-Ala, Gly-Gly-Phe-Gly (GGFG), MAC glucuronide phenol.

**7.** The antibody-drug conjugate or salt thereof according to any one of claims 1 to 6, wherein $L_2$-CTD is VcMMAE, GGFG-Dxd or VC-seco-DUBA;

preferably, in case that Ar' is a substituted or unsubstituted 5-12 membered heteroarylene, the heteroatom is N;
preferably, Ar' is a substituted or unsubstituted C6 arylene or a substituted or unsubstituted 6 membered heteroarylene;

more preferably, the antibody-drug conjugate or salt thereof has the structure as follows:

Conjugate ADC-1:

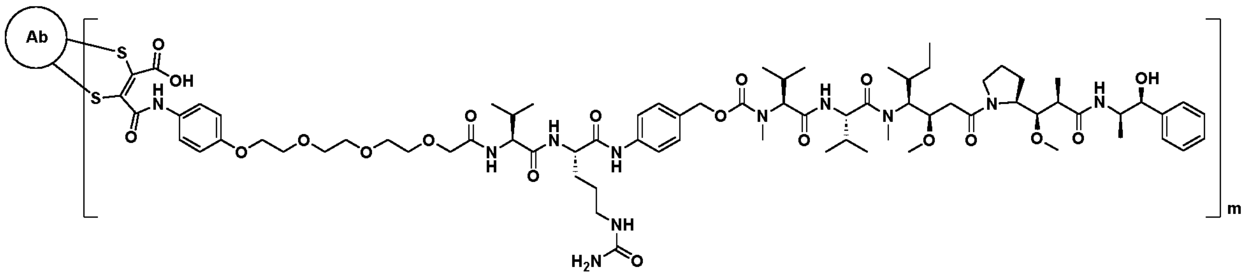

Conjugate ADC-2:

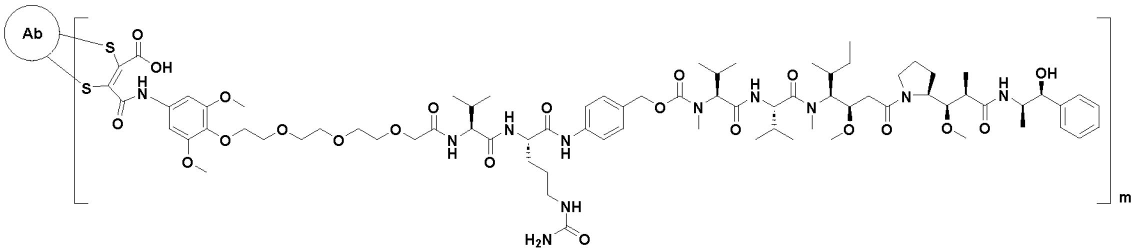

Conjugate ADC-3 :

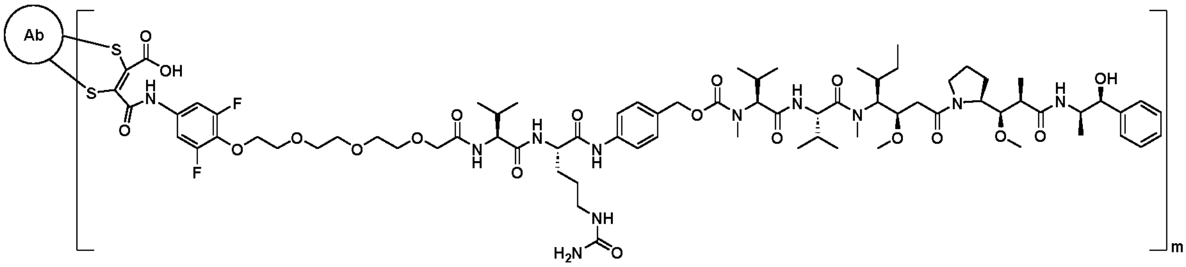

Conjugate ADC-4:

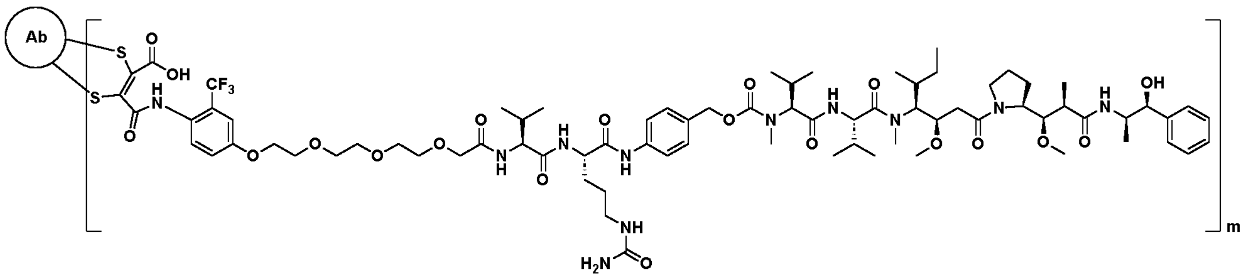

Conjugate ADC-5:

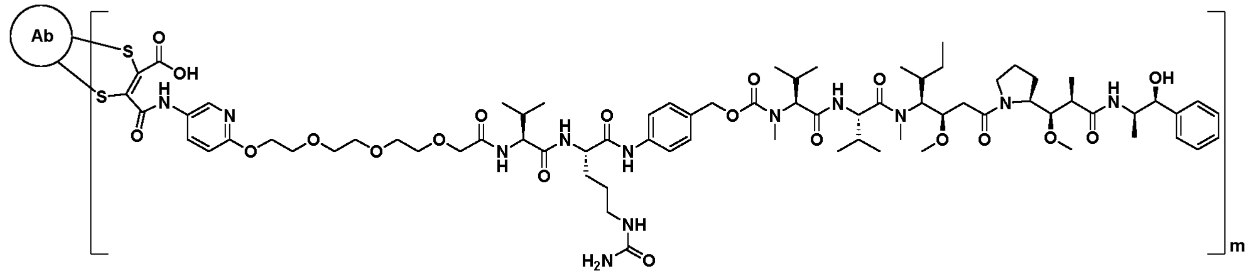

Conjugate ADC-6:

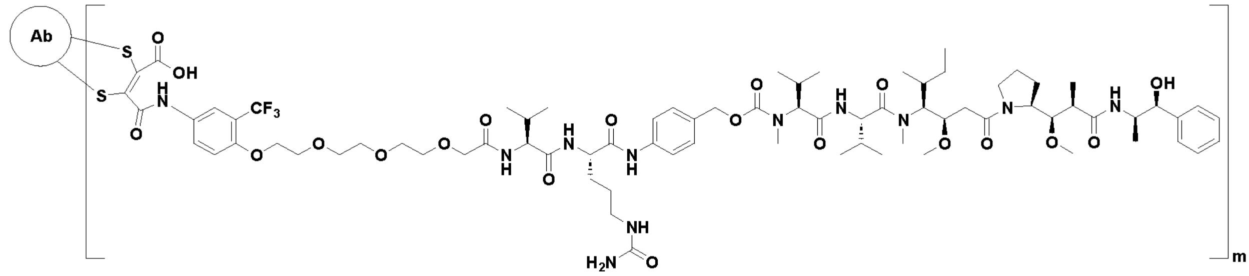

Conjugate ADC-7:

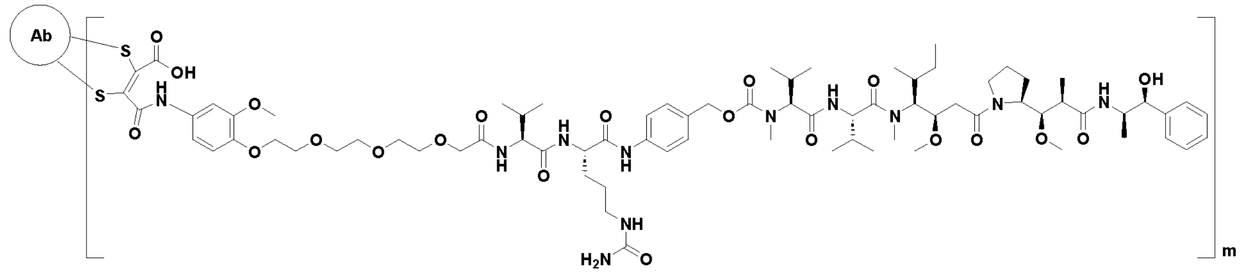

**8.** A preparation method for an antibody-drug conjugate targeting Nectin-4 or salt thereof, wherein the antibody-drug conjugate or salt thereof has a structure represented by the formula Ia and/or Ib as follows:

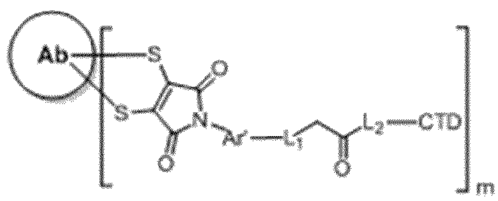

Ia

and/or

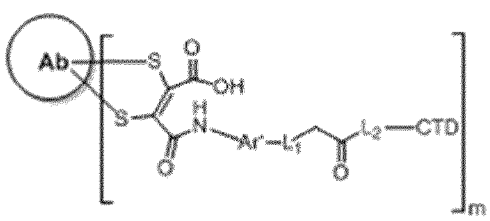

Ib

the method comprising the following steps:

(1) reacting the anti-Nectin-4 antibody or fragment thereof with a reducing agent in a buffer, to obtain a reduced antibody or fragment thereof;
(2) conjugating a drug-linker (a linker-drug conjugate) to the reduced antibody or fragment thereof obtained in step (1) in a mixture of a buffer and an organic solvent, to obtain the antibody-drug conjugate targeting Nectin-4.

**9.** The preparation method according to claim 8, wherein the drug-linker has a structure represented by the formula Ic as follows:

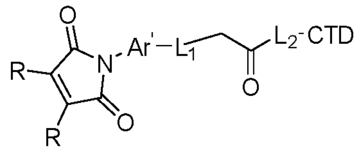

Ic

wherein:

R is X or R'S, wherein X is halogen (F, Cl, Br or I), preferably Br or I; R' is substituted or unsubstituted C6-C10 aryl or substituted or unsubstituted 5-12 membered heteroaryl, wherein the substitution refers to the replacement of a hydrogen atom on a group by one or more substituents selected from the group consisting of: alkyl (e.g., C1-C6 alkyl, preferably C1-C4 alkyl), alkoxy (e.g., C1-C6 alkoxy, preferably C1-C4 alkoxy, e.g. methoxy), halogen (F, Cl, Br or I), ester, amide and cyano;
preferably, R is R'S, wherein R' is phenyl or substituted phenyl, and the substituent in the substituted phenyl is selected from the group consisting of alkyl (e.g., C1-C6 alkyl, preferably C1-C4 alkyl), alkoxy (e.g., C1-C6 alkoxy, preferably C1-C4 alkoxy, more preferably methoxy), halogen (F, Cl, Br or I), ester, amide and cyano; preferably, R' is phenyl, 4-methylformamido-substituted phenyl

( 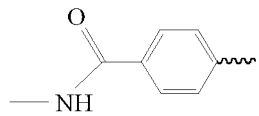 ),

or 4-formylmorpholine-substituted phenyl

( 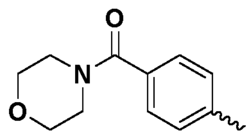 );

and
Ar', $L_1$, $L_2$ and CTD are as defined in any one of claims 6 to 8.

**10.** The preparation method according to claim 8 or 9, wherein the drug-linker is any one selected from the group consisting of:

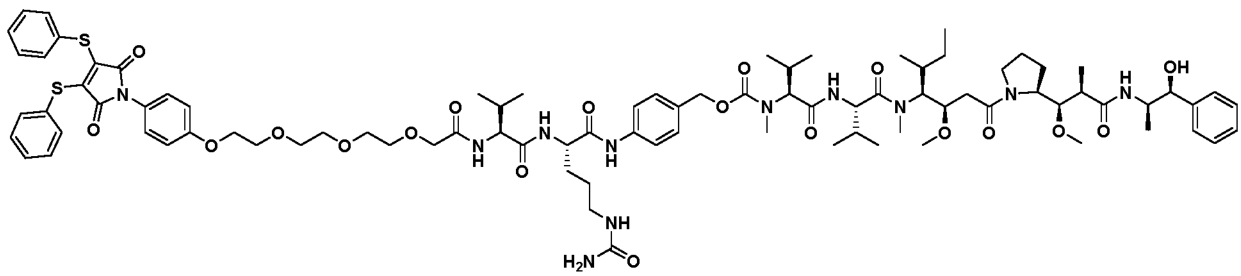

A-1

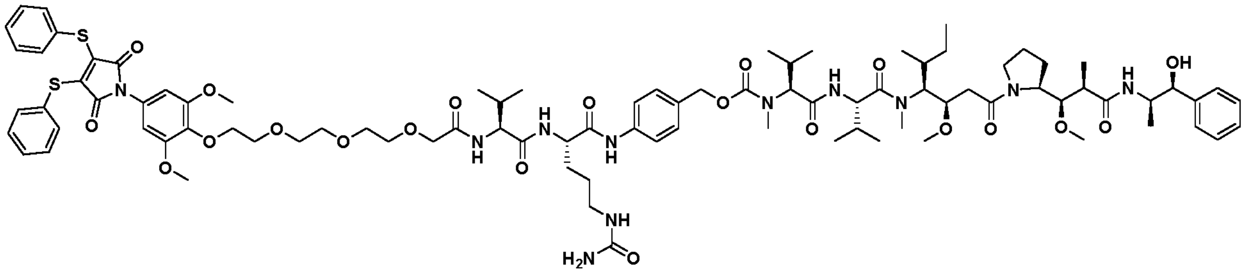

A-2

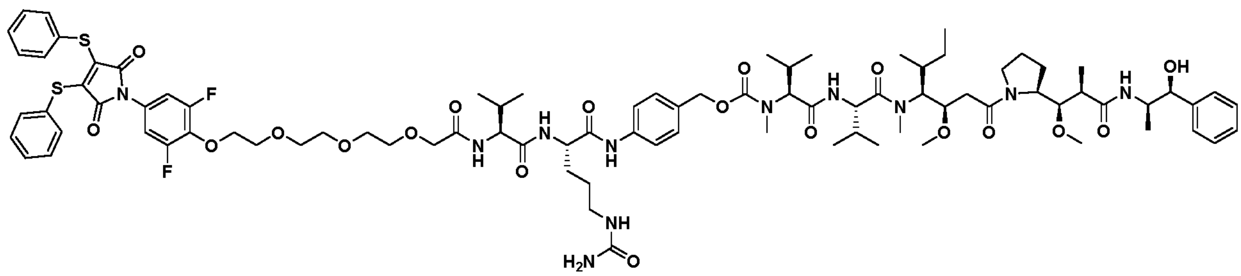

A-3

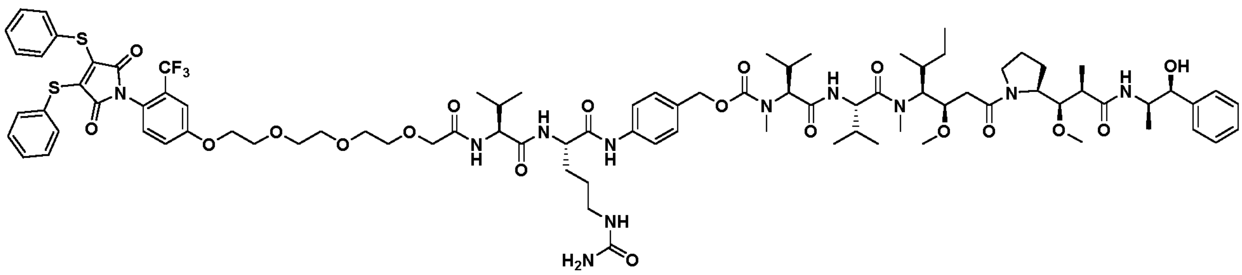

A-4

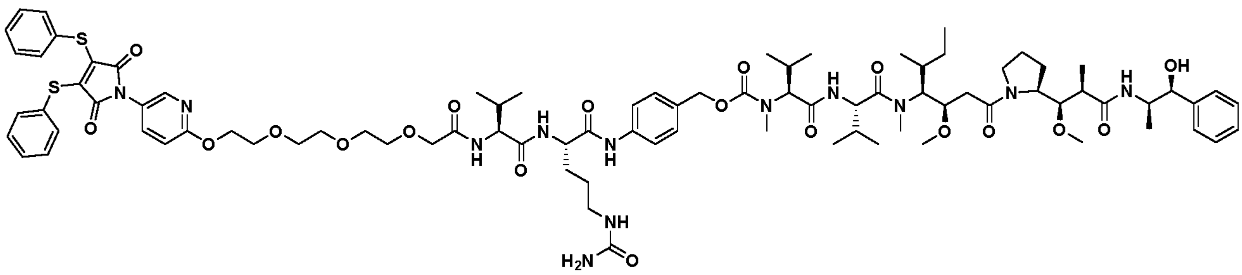

A-5

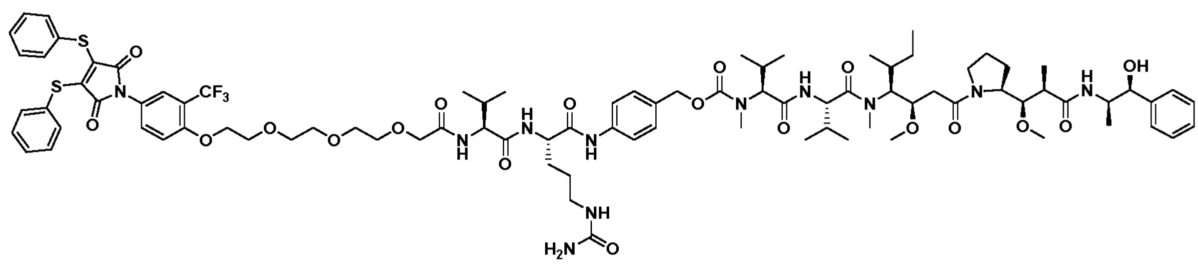

A-6

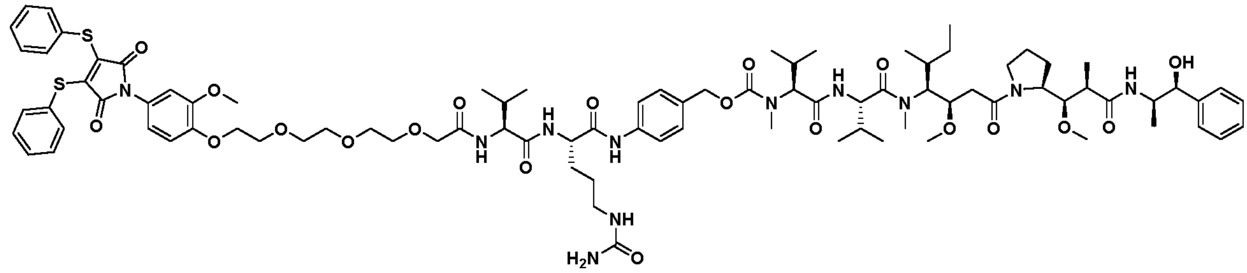

A-7

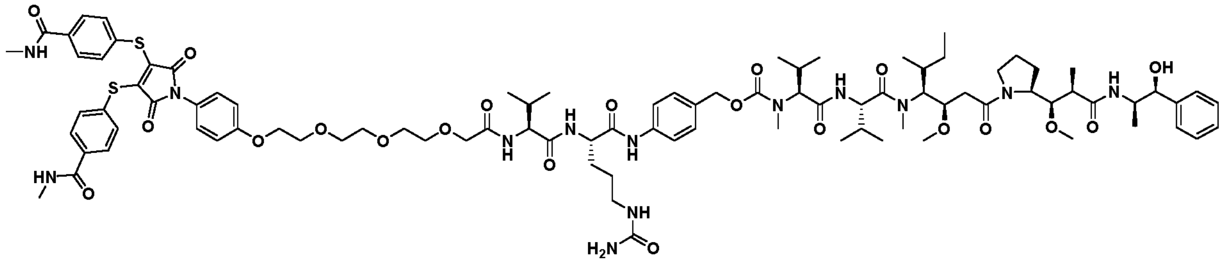

B-1

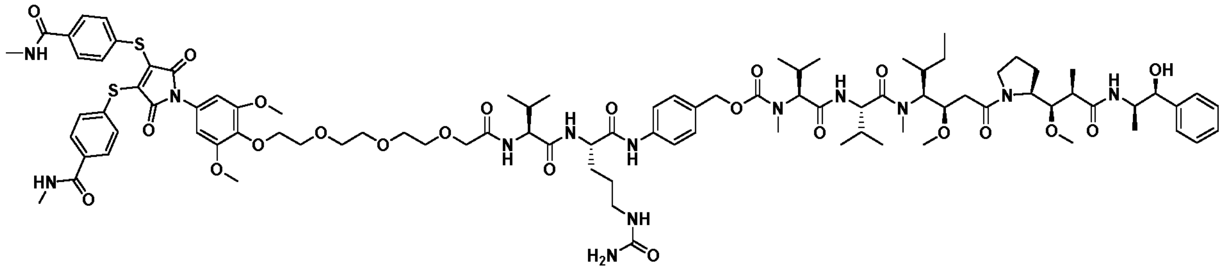

B-2

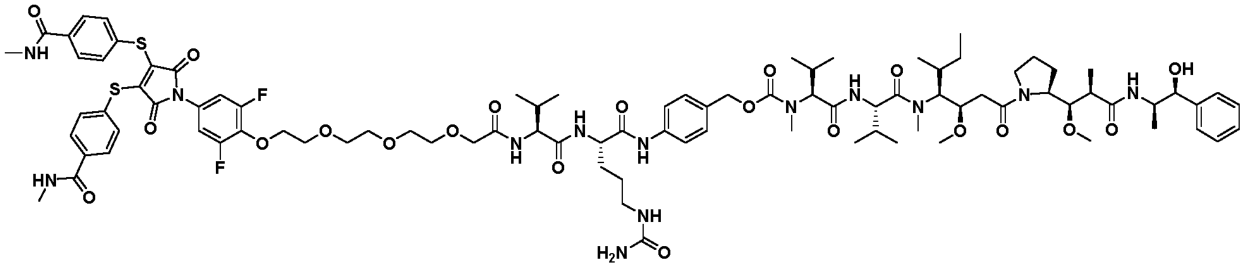

B-3

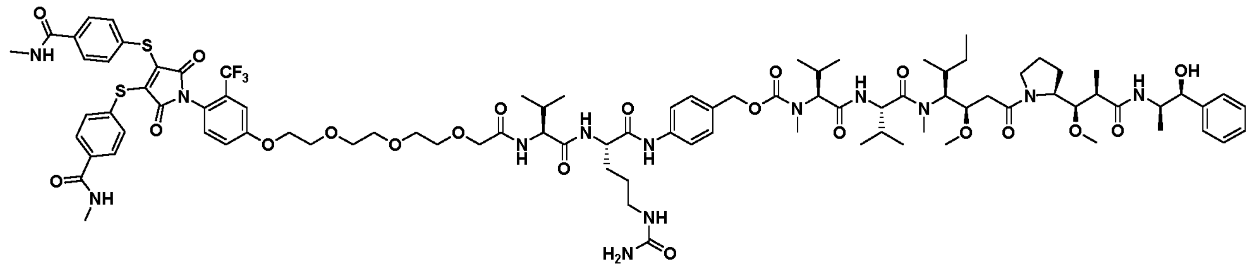
B-4

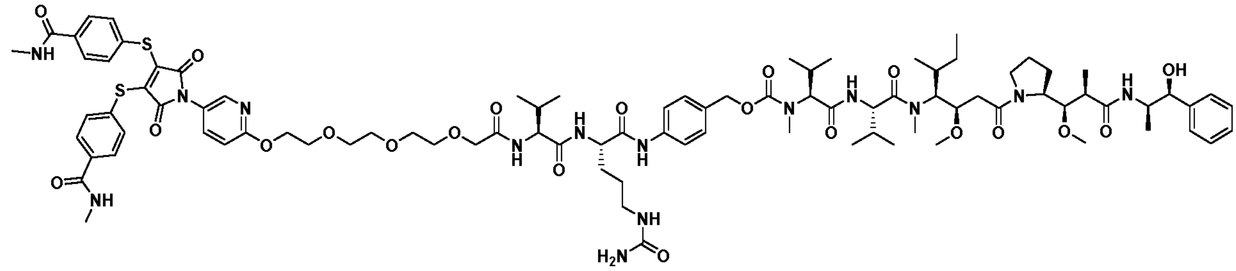
B-5

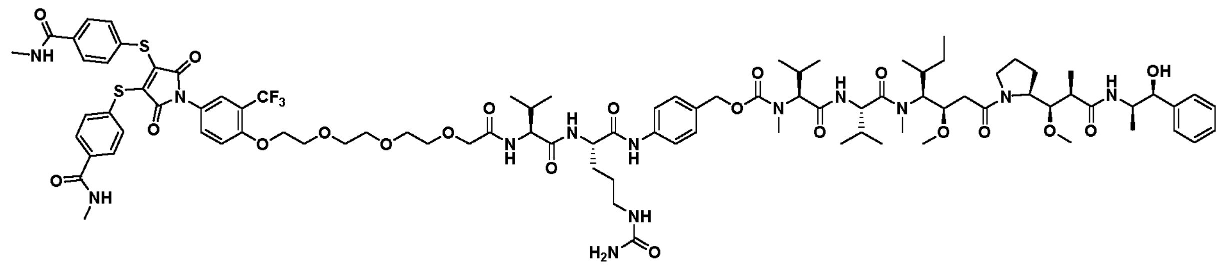
B-6

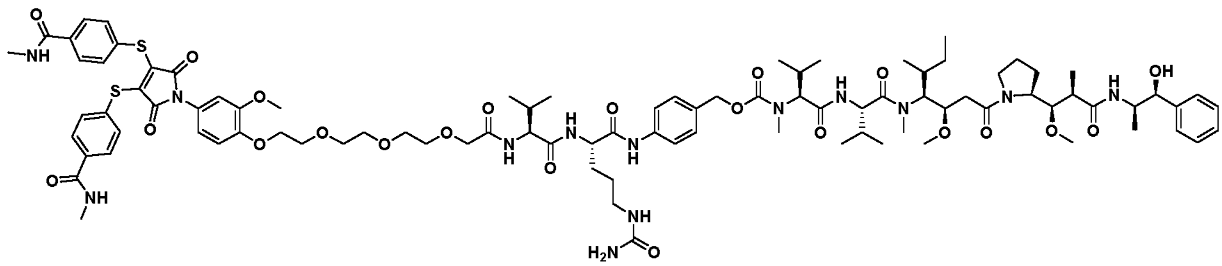
B-7

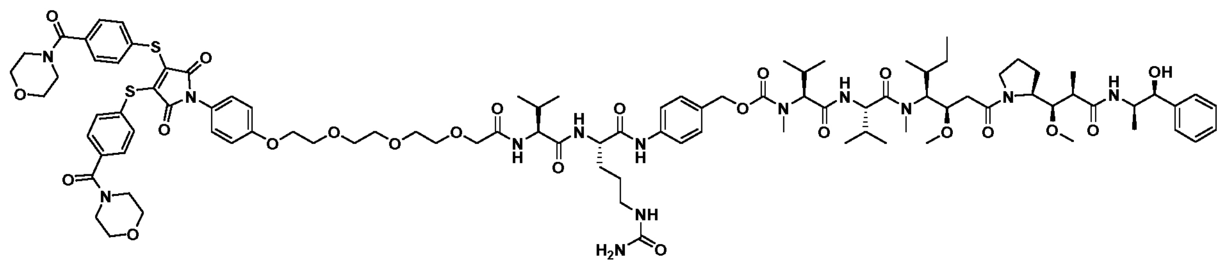
C-1

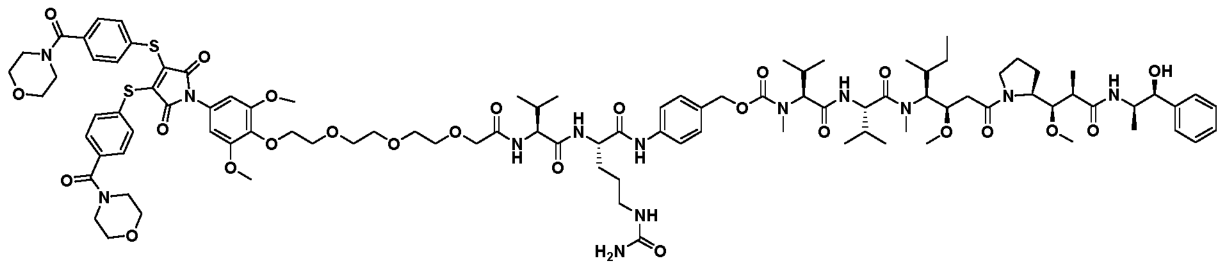

C-2

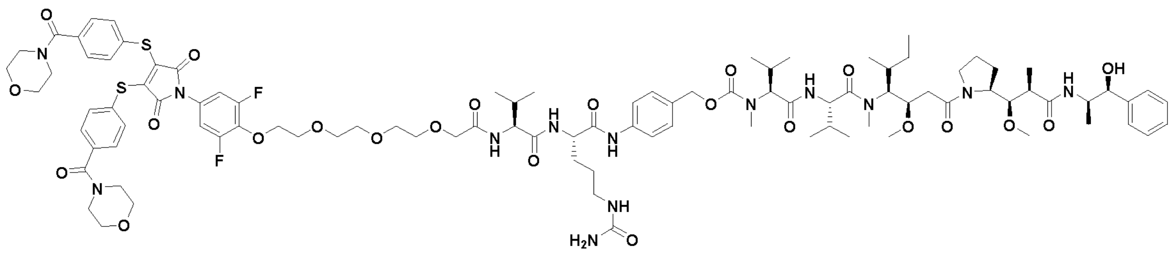

C-3

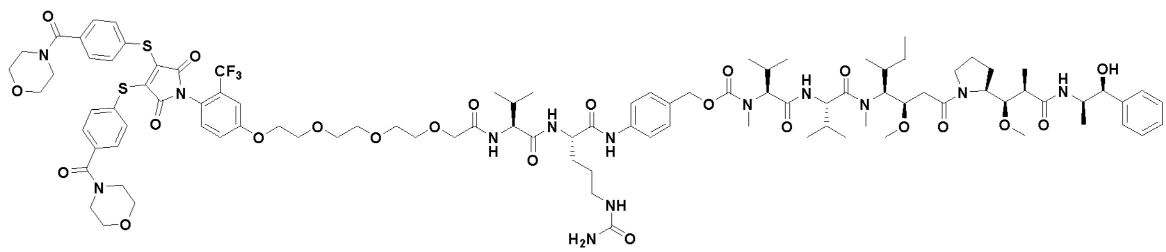

C-4

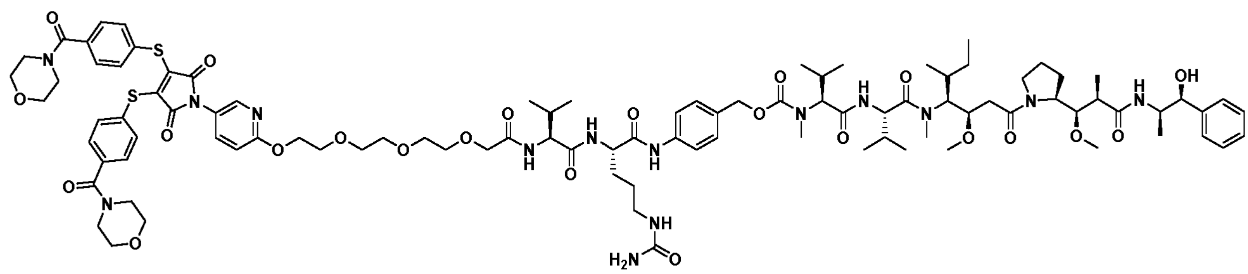

C-5

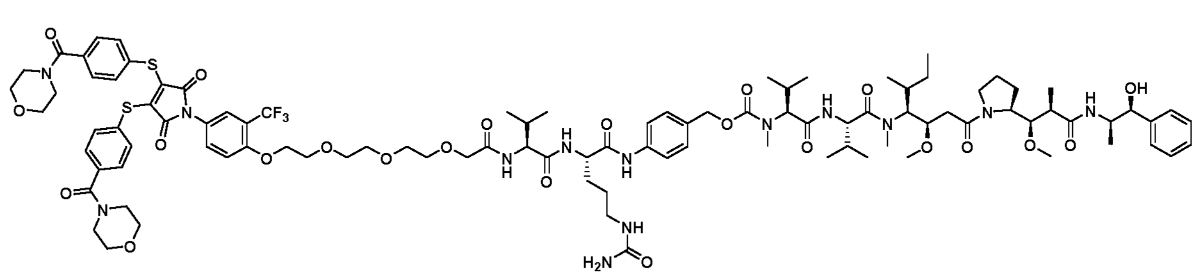

C-6

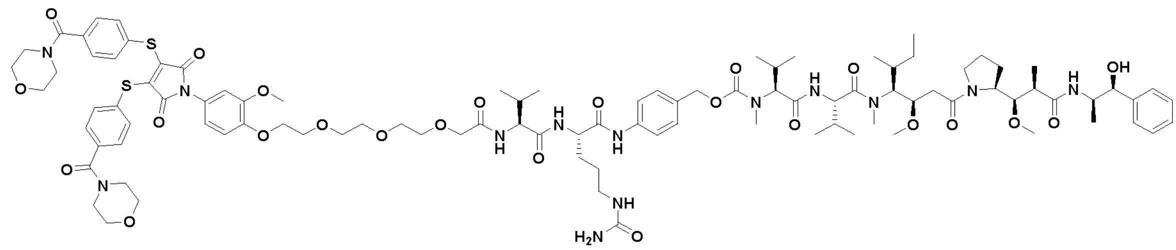

C-7

**11.** The preparation method according to any one of claims 8 to 10, wherein the preparation method comprises the following steps:

a. Antibody reduction: adding a reducing agent to a phosphate buffer containing the antibody in a concentration of 5-30 mg/mL at an equivalent molar ratio of ≥ 5.5: 1 (the reducing agent: the antibody), and reacting the reducing agent and the antibody for 1.5-2 hours, wherein the reducing agent is one or more selected from the group consisting of TCEP, DTT, 2-MEA, and DTBA;
b. Antibody conjugation: displacing the reduced antibody obtained in step a into a phosphate buffer at pH 6.5-7.8, thereby diluting the antibody to a concentration of 3.5-15 mg/mL in the buffer to obtain a diluted antibody solution; adding a drug-containing linker dissolved in an organic co-solvent to the diluted antibody solution at an equivalent molar ratio of 4.5-6.5: 1 (the drug-containing linker: the antibody), and then reacting the reaction system under stirring at 15-35 °C for ≥ 0.5 h, wherein the organic co-solvent is one or more selected from the group consisting of DMA, DMSO, DMF, and ACN;
c. Hydrophobic chromatography: subjecting the antibody conjugation product obtained to purification through hydrophobic chromatography using hydrophobic filler;

preferably, the preparation method further comprises the following step after step b or after step c:
d. Hydrolysis: displacing the antibody conjugation product into a phosphate buffer at pH 7.4-9.0, and then heating the buffer at 35±10 °C for 2-24 hours to obtain a hydrolysis product.

**12.** An anti-Nectin-4 antibody or fragment thereof comprising a heavy chain and a light chain, wherein the heavy chain and the light chain comprise heavy chain complementarity determining regions 1 to 3 (CDR-H1, CDR-H2 and CDR-H3) and light chain complementarity determining regions 1 to 3 (CDR-L1, CDR-L2 and CDR-L3) respectively as follows:
CDR-H1, CDR-H2 and CDR-H3 having amino acid sequences as shown in SEQ ID NO: 19, SEQ ID NO: 20 and SEQ ID NO: 13 respectively; and, CDR-L1, CDR-L2 and CDR-L3 having amino acid sequences as shown in SEQ ID NO: 14, SEQ ID NO: 15 and SEQ ID NO: 16 respectively.

**13.** The anti-Nectin-4 antibody or fragment thereof according to claim 12, wherein the heavy chain and the light chain comprised in the anti-Nectin-4 antibody or fragment thereof comprise a heavy chain variable region (VH) and a light chain variable region (VL), and wherein the heavy chain variable region (VH) comprises an amino acid sequence as shown in SEQ ID NO: 5 or SEQ ID NO: 7 or a variant thereof, and the light chain variable region (VL) comprises an amino acid sequence as shown in SEQ ID NO: 6 or SEQ ID NO: 8 or a variant thereof;

preferably, the heavy chain variable region (VH) and the light chain variable region (VL) of the anti-Nectin-4 antibody or fragment thereof comprise:

(A) the amino acid sequence as shown in SEQ ID NO: 5 or a variant thereof; and, the amino acid sequence as shown in SEQ ID NO: 6 or a variant thereof; or
(B) the amino acid sequence as shown in SEQ ID NO: 7 or a variant thereof; and, the amino acid sequence as shown in SEQ ID NO: 8 or a variant thereof;

further preferably, the anti-Nectin-4 antibody or fragment thereof is as defined in claim 4 or 5.

**14.** A nucleic acid molecule comprising a nucleotide sequence encoding a heavy chain variable region, a light chain variable region, a heavy chain or a light chain comprised in the anti-Nectin-4 antibody or fragment thereof according to claim 12 or 13.

**15.** A vector comprising the nucleic acid molecule according to claim 14.

**16.** A host cell comprising the nucleic acid molecule according to claim 14 and/or the vector according to claim 15.

**17.** A composition comprising the antibody-drug conjugate targeting to Nectin-4 or salt thereof according to any one of claims 1 to 7, the anti-Nectin-4 antibody or fragment thereof according to claim 12 or 13, the nucleic acid molecule according to claim 14, the vector according to claim 15, and/or the host cell according to claim 16.

**18.** Use of the antibody-drug conjugate targeting to Nectin-4 or salt thereof according to any one of claims 1 to 7, the anti-Nectin-4 antibody or fragment thereof according to claim 12 or 13, the nucleic acid molecule according to claim 14, the vector according to claim 15, the host cell according to claim 16, and/or the composition according to claim 17 in the manufacture of a medicament for treating a tumor.

**19.** The use according to claim 18, wherein the tumor is a tumor or cancer associated with high expression of Nectin-4; preferably, the tumor or cancer is any one selected from the group consisting of: bladder cancer, breast cancer, ovarian cancer, pancreatic cancer, hepatocellular cancer, gastric cancer, non-hodgkin's lymphoma, hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, colorectal cancer, glioma, and mesothelioma.

**20.** A method for treating a tumor, comprising administering to a subject in need thereof the antibody-drug conjugate targeting to Nectin-4 or salt thereof according to any one of claims 1 to 7, the anti-Nectin-4 antibody or fragment thereof according to claim 12 or 13, the nucleic acid molecule according to claim 14, the vector according to claim 15, the host cell according to claim 16, and/or the composition according to claim 17.

**21.** The method according to claim 20, wherein the subject is a mammal, preferably a primate, more preferably a human;

preferably, the tumor is a tumor or cancer associated with high expression of Nectin-4;
preferably, the tumor or cancer is any one selected from the group consisting of: bladder cancer, breast cancer, ovarian cancer, pancreatic cancer, hepatocellular cancer, gastric cancer, non-hodgkin's lymphoma, hodgkin's lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, colorectal cancer, glioma, and mesothelioma.

**FIG. 1**

**1A**

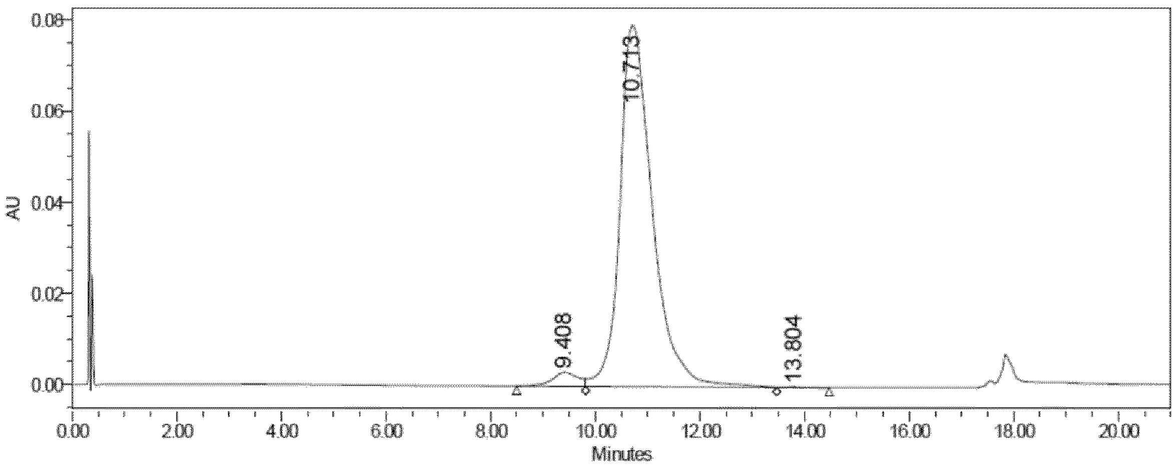


| Time (min) | Area | Percent area (%) | Peak height |
|---|---|---|---|
| 9.408 | 105247 | 3.00 | 3102 |
| 10.713 | 3404231 | 96.91 | 79100 |
| 13.804 | 3211 | 0.09 | 98 |

1B

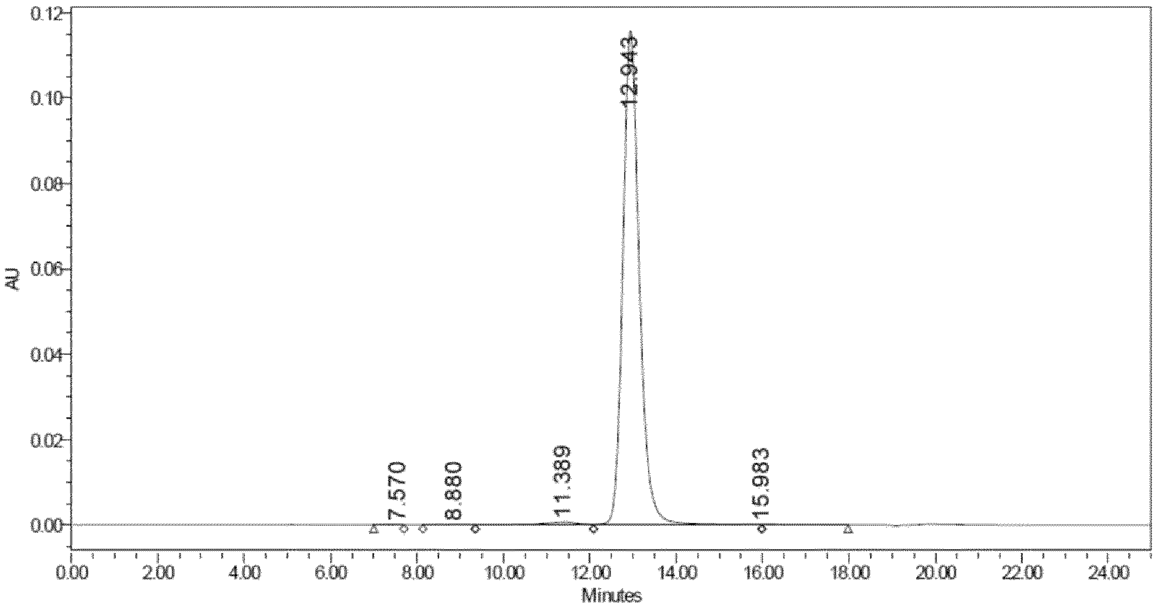


| Time (min) | Area | Percent area (%) | Peak height |
|---|---|---|---|
| 7.570 | 339 | 0.01 | 49 |
| 8.880 | 1853 | 0.06 | 34 |
| 11.389 | 34848 | 1.12 | 662 |
| 12.943 | 3058044 | 98.67 | 115675 |
| 15.983 | 4244 | 0.14 | 78 |

1C

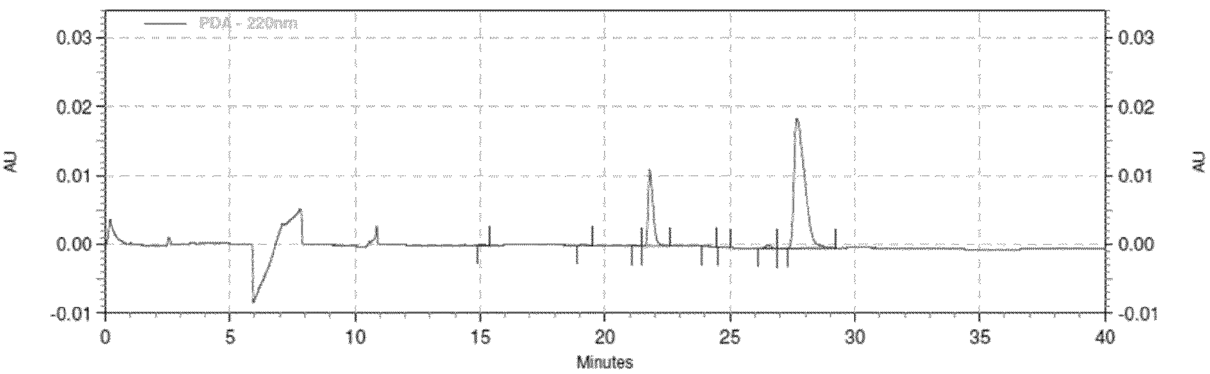


| Time (min) | Area | Percent area (%) |
|---|---|---|
| 15.025 | 35 | 0.39 |
| 19.100 | 15 | 0.16 |
| 21.342 | 15 | 0.17 |
| 21.808 | 2278 | 25.21 |
| 24.017 | 9 | 0.10 |
| 24.775 | 14 | 0.16 |
| 26.533 | 89 | 0.99 |
| 27.183 | 13 | 0.14 |
| 27.692 | 6564 | 72.67 |

1D

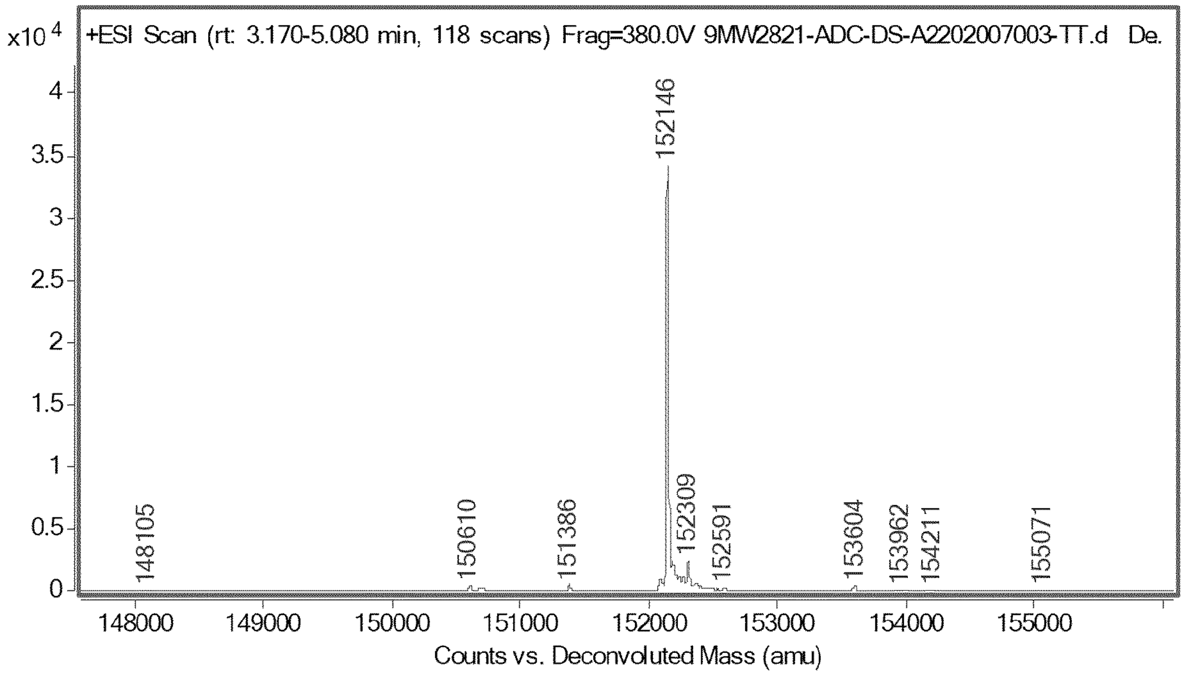


| Dn | Measure molecular weight (Da) | Height | Area | Ratio-H | Ratio-A |
|---|---|---|---|---|---|
| D3 | 150610 | 444.62 | 9283 | 0.012 | 0.013 |
| D3+suger | 150772 | 50.61 | 1740 | 0.001 | 0.003 |
| D4 | 152146 | 34221.09 | 614335 | 0.920 | 0.893 |
| D4+suger | 152309 | 2478.63 | 62571 | 0.067 | 0.091 |
| Sum | | 37194.95 | 687929 | 1.000 | 1.000 |
| DAR | | | | 3.99 | 3.98 |

FIG. 2

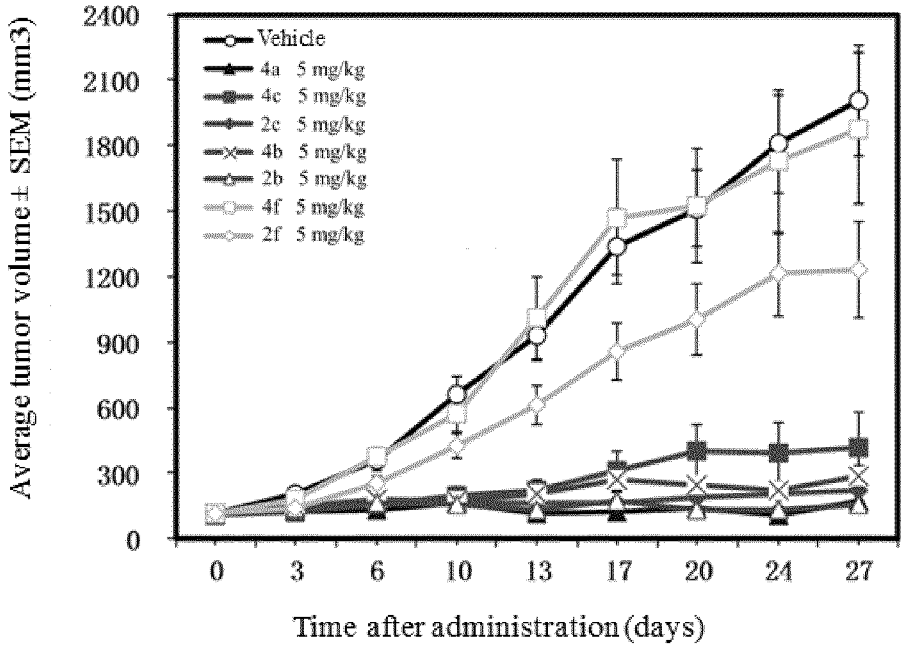


FIG. 3

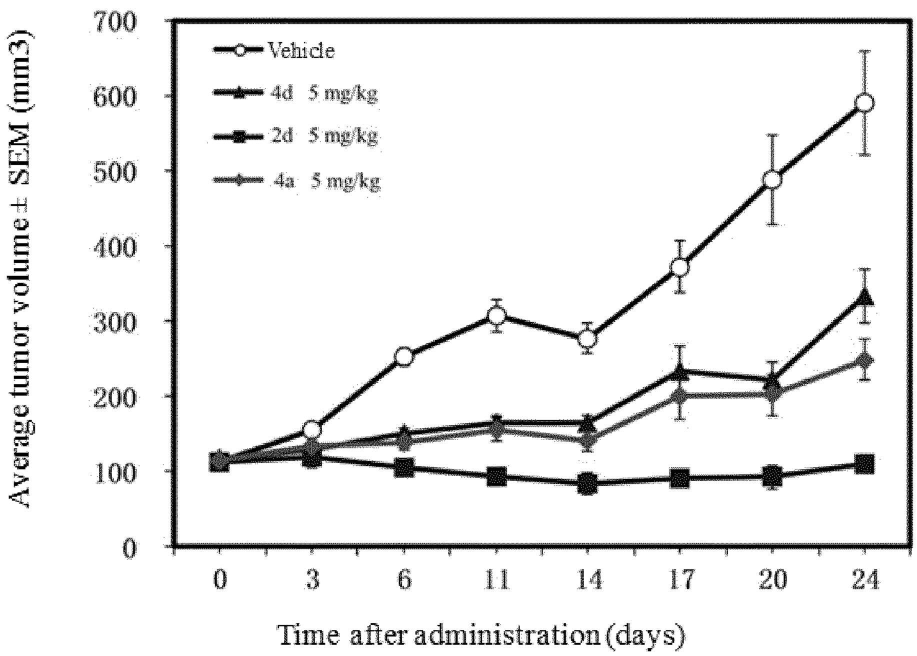


FIG. 4

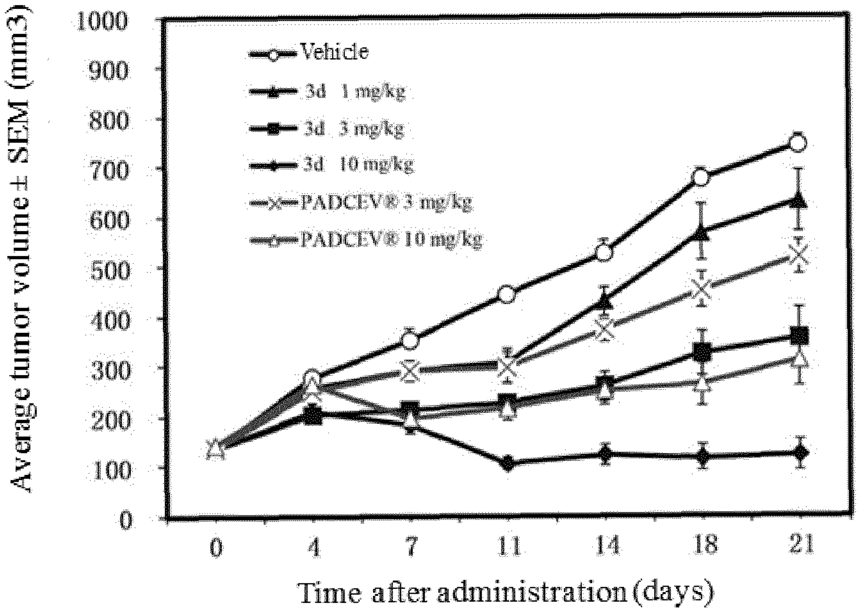
Vehicle
3d 1 mg/kg
3d 3 mg/kg
3d 10 mg/kg
PADCEV® 3 mg/kg
PADCEV® 10 mg/kg
Average tumor volume ± SEM (mm3)
1000
900
800
700
600
500
400
300
200
100
0
0
4
7
11
14
18
21
Time after administration (days)

FIG. 5

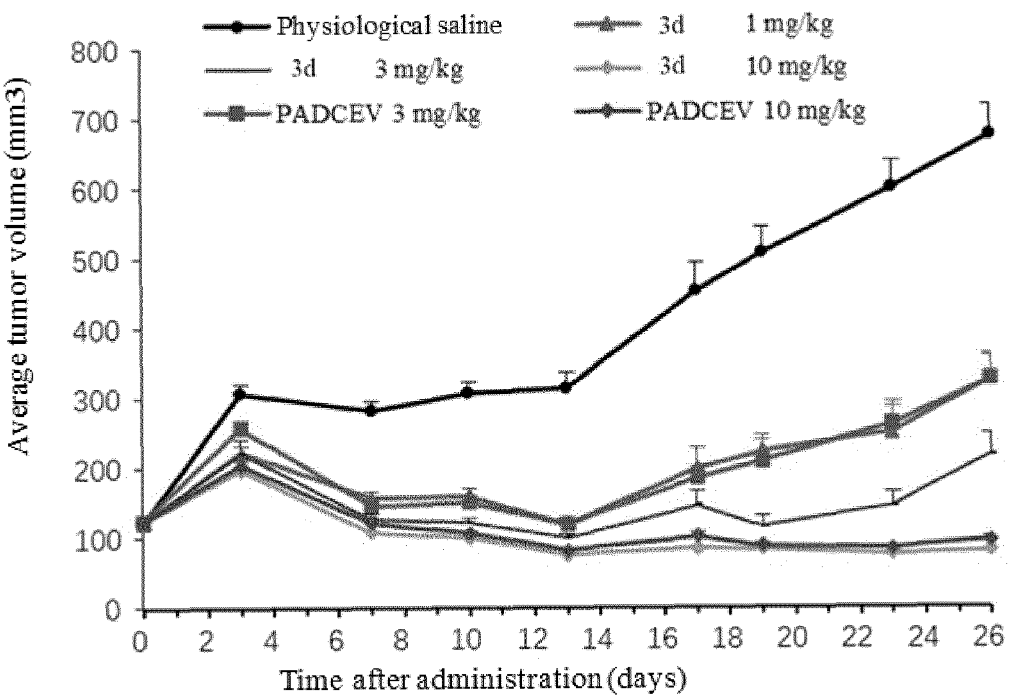
Physiological saline
3d 1 mg/kg
3d 3 mg/kg
3d 10 mg/kg
PADCEV 3 mg/kg
PADCEV 10 mg/kg
Average tumor volume (mm3)
800
700
600
500
400
300
200
100
0
0 2 4 6 8 10 12 14 16 18 20 22 24 26
Time after administration (days)

FIG. 6

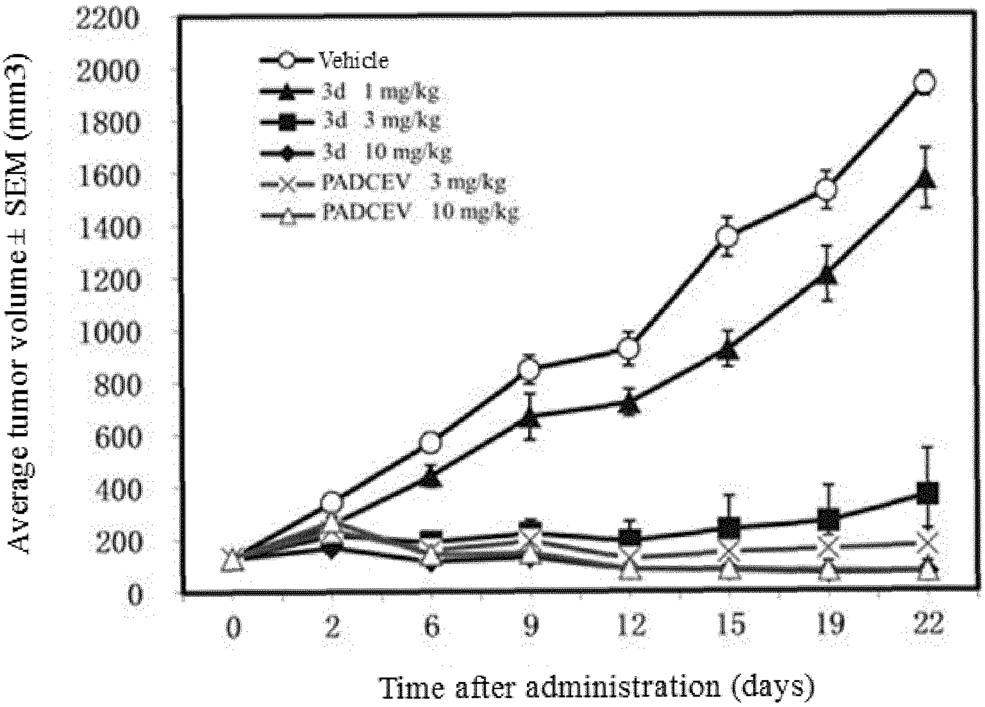
2200
2000
1800
1600
1400
1200
1000
800
600
400
200
0
Average tumor volume± SEM (mm3)
Vehicle
3d 1 mg/kg
3d 3 mg/kg
3d 10 mg/kg
PADCEV 3 mg/kg
PADCEV 10 mg/kg
0
2
6
9
12
15
19
22
Time after administration (days)

**INTERNATIONAL SEARCH REPORT**

International application No.
**PCT/CN2022/090450**

**A. CLASSIFICATION OF SUBJECT MATTER**

A61K 47/68(2017.01)i； C07K 16/28(2006.01)i； A61P 35/00(2006.01)i

According to International Patent Classification (IPC) or to both national classification and IPC

**B. FIELDS SEARCHED**

Minimum documentation searched (classification system followed by classification symbols)

A61K; C07K; A61P

Documentation searched other than minimum documentation to the extent that such documents are included in the fields searched

Electronic data base consulted during the international search (name of data base and, where practicable, search terms used)

CNABS, SIPOABS, DWPI, CNTXT, EPTXT, WOTXT, USTXT, CNKI, ISI web of science , baidu, pubmed, STN, 江苏迈威康新药研发有限公司, 周伟, Nectin-4, 抗体, 缀合物, 偶联物, conjugate, SEQ ID NOs: 1-20

**C. DOCUMENTS CONSIDERED TO BE RELEVANT**

| Category* | Citation of document, with indication, where appropriate, of the relevant passages | Relevant to claim No. |
|---|---|---|
| PX | CN 113527486 A (MABWELL (SHANGHAI) BIOSCIENCE CO., LTD.) 22 October 2021 (2021-10-22)<br>entire document | 1-21 |
| A | WO 2021069508 A1 (Aix-Marseille University et al.) 15 April 2021 (2021-04-15)<br>entire document | 1-21 |
| A | CN 109810039 A (SHANGHAI QINGRUN PHARMACEUTICAL TECHNOLOGY CO., LTD.) 28 May 2019 (2019-05-28)<br>entire document | 1-21 |
| A | WO 2017042210 A1 (INSERM INSTITUT NAT DE LA SANTÉ ET DE LA RECHERCHE MÉDICALE et al.) 16 March 2017 (2017-03-16)<br>entire document | 1-21 |
| A | WO 2019243832 A1 (BICYCLETX LIMITED) 26 December 2019 (2019-12-26)<br>entire document | 1-21 |
| A | CHALLITA-EID, P. M. et al. "Enfortumab Vedotin Antibody–Drug Conjugate Targeting Nectin-4 Is a Highly Potent Therapeutic Agent in Multiple Preclinical Cancer Models." *Cancer Research.*, Vol. 76, No. 10, 24 March 2016 (2016-03-24),<br>pp. 3003-3013 | 1-21 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 July 2022** | **27 July 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

INTERNATIONAL SEARCH REPORT

International application No.
**PCT/CN2022/090450**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2022/090450**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **20-21**
because they relate to subject matter not required to be searched by this Authority, namely:

[1] Claims 20-21 relate to a method for treating tumors, which belongs to a method for treating diseases as defined in PCT Rule 39.1(iv). However, a search was still conducted on the basis of a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/090450**

| Patent document cited in search report | | | Publication date (day/month/year) | Patent family member(s) | | | Publication date (day/month/year) |
|---|---|---|---|---|---|---|---|
| CN | 113527486 | A | 22 October 2021 | None | | | |
| WO | 2021069508 | A1 | 15 April 2021 | IL | 291966 | D0 | 01 June 2022 |
| | | | | AU | 2020364959 | A1 | 07 April 2022 |
| | | | | CA | 3156451 | A1 | 15 April 2021 |
| CN | 109810039 | A | 28 May 2019 | None | | | |
| WO | 2017042210 | A1 | 16 March 2017 | JP | 2018531913 | A | 01 November 2018 |
| | | | | US | 2018243434 | A1 | 30 August 2018 |
| | | | | EP | 3347048 | A1 | 18 July 2018 |
| | | | | ES | 2794557 | T3 | 18 November 2020 |
| WO | 2019243832 | A1 | 26 December 2019 | PH | 12020552179 | A1 | 28 June 2021 |
| | | | | CN | 112601539 | A | 02 April 2021 |
| | | | | KR | 20210029783 | A | 16 March 2021 |
| | | | | TW | 202016129 | A | 01 May 2020 |
| | | | | WO | 2019243833 | A1 | 26 December 2019 |
| | | | | BR | 112020025985 | A2 | 23 March 2021 |
| | | | | IL | 279489 | D0 | 31 January 2021 |
| | | | | JP | 2021527701 | A | 14 October 2021 |
| | | | | SG | 11202012413 T | A | 28 January 2021 |
| | | | | US | 2022089643 | A1 | 24 March 2022 |
| | | | | AU | 2019289199 | A1 | 11 February 2021 |
| | | | | EP | 3810167 | A1 | 28 April 2021 |
| | | | | US | 2019389906 | A1 | 26 December 2019 |
| | | | | KR | 20210025603 | A | 09 March 2021 |
| | | | | EP | 3810169 | A1 | 28 April 2021 |
| | | | | CN | 112566651 | A | 26 March 2021 |
| | | | | CA | 3101427 | A1 | 26 December 2019 |
| | | | | IL | 279488 | D0 | 31 January 2021 |
| | | | | BR | 112020025715 | A2 | 06 April 2021 |
| | | | | SG | 11202012415 P | A | 28 January 2021 |
| | | | | CA | 3101164 | A1 | 26 December 2019 |
| | | | | AU | 2019289200 | A1 | 11 February 2021 |
| | | | | JP | 2021527702 | A | 14 October 2021 |
| | | | | US | 2021269480 | A1 | 02 September 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110481199
- WO 2018095422 A1
- CN 202011046911X
- US 20090010945 A1


### Non-patent literature cited in the description


- *Clin Cancer Res.,* 15 October 2004, vol. 10 (20), 7063-70